# EUROPEAN PATENT APPLICATION

(11) **EP 4 840 503 A2**
(43) Date of publication of application: **18.11.2026**
(21) Application number: 26200706.5
(22) Date of filing: 10.09.2019
(51) Int. Cl.: A61N 5/06

(54) **LASER SYSTEM AND METHOD FOR OPERATING THE LASER SYSTEM**

(62) Document number(s) of the earlier application(s) in accordance with Art. 76 EPC: 19196370.1 / 3 791 821
(71) Applicant: Fotona d.o.o., 1000 Ljubljana (SI)
(72) Inventor: LUKAC, Matjaz, 1000 Ljubljana (SI); LUKAC, Nejc, 1000 Ljubljana (SI); JEZERSEK, Matija, 1235 Radomlje (SI)
(74) Representative: Bardehle Pagenberg Partnerschaft mbB Patentanwälte Rechtsanwälte

(57) **Abstract**

An apparatus and a method for cleaning a cavity filled with a liquid are disclosed. An apparatus (1) for applying pulses of electromagnetic radiation to a cavity (2) filled with a liquid (3) may comprise a source (4, 4') for generating a first pulse and a second pulse of electromagnetic radiation and a control unit (22) adapted to control a time between the first pulse and the second pulse as a function of a diameter D and/or a cross-sectional area of the cavity (2).

## Description

### TECHNICAL FIELD

The present invention generally relates to an apparatus for cleaning a cavity filled with a liquid (e.g., debridement, material removal, irrigation, disinfection, decontamination of surfaces of the cavity, and/or for fragmenting particles within such cavities) and corresponding methods.

### BACKGROUND

When energy is locally deposited within a liquid, for example with intense focused electromagnetic radiation (e.g., laser light) or with an electrical discharge through a spark, locally induced boiling of the liquid leads to a creation of a cavitation bubble that rapidly expands due to the high pressure within the vapor. When the bubble reaches its maximum volume where the internal pressure is lower than in the surrounding liquid, the bubble starts to collapse. When the collapsing bubble reaches a given size, it may rebound, and the process repeats until there is insufficient energy for the bubble to rebound again. These violent cavitation oscillations lead to rapid streaming of liquid molecules around the cavitation bubble. It is also known that a cavitation bubble collapsing near a boundary forms a liquid jet directed at the boundary. Even more importantly, under appropriate conditions, an intense shock wave may be emitted during the bubble's collapse.

The strong mechanical forces associated with rapid bubble oscillations can break particles or remove particles from surfaces, thus locally cleaning them. This effect is of interest for industrial applications, and as well in medicine. Laser-induced cavitation bubbles have been used in ophthalmology, cardiology, urology and dentistry. For example, laser pulses produce plasma with subsequent bubble formation for ocular surgery by photo-disruption. Laser induced lithotripsy fragments kidney stones through cavitation erosion. Laser pulses have been used to remove thrombus in obstructed arteries. In endodontics, laser activated irrigation is used to debride dental root canals. Laser induced cavitation may be also used for cleaning (e.g., debriding and disinfection) of periodontal pockets, holes created during bone surgery, or surfaces of inserted implants.

The principle lying behind cavitation phenomena is the difference in compressibility between a gas and a liquid. The volume of liquid hardly changes in response to a variation in pressure, whereas the volume of the gaseous interior of a bubble can change dramatically. Any contraction or expansion of the bubble is inevitably accompanied by a displacement of an equal volume of the much denser surrounding liquid. As a result, a strong bubble response in combination with the compressible interior can provide not only localized fluid motion but also tremendous focusing of the liquid kinetic energy. Of particular interest for cleaning are the shock waves which may form during the bubble's collapse. These shock waves spread through the volume at supersonic speeds, and interact disruptively with the surrounding environment (e.g., cavity walls). These waves are not only very effective in removing any contamination from the cavity surfaces but can also kill bacteria, leading to a partial or complete disinfection of the cavity.

In an infinite liquid, a secondary shock wave is emitted during the accelerated contraction of the bubble cavity. This secondary shock wave is to be distinguished from the primary shock wave which is sometimes emitted during the initial bubble expansion phase when laser energy is locally deposited into a liquid within a very short time of nanoseconds or less. In what follows, the term "shock wave" will represent the secondary shock wave emission only.

The (secondary) shock wave emission occurs as follows. At the initial moment of the bubble's contraction, the pressure inside the bubble equals that of the saturated vapor which is much less than the liquid pressure. Because of this transition, the bubble starts to contract, and the bubble vapor pressure starts to grow. Initially, the bubble contraction is relatively slow. However, as the pressure rises, this leads to a vapor mass loss due to the condensation process on the bubble surface, accelerating the implosion even further. This ever-faster acceleration results in a violent collapse of the bubble, leading to heating up of the vapor and, most importantly, to emission of a supersonic shock wave emanating from the collapsed bubble. And finally, when the vapor temperature reaches its critical value the condensation process stops, which leads to an even faster rise of the vapor pressure until the contraction stops and the bubble begins to rebound.

Whether the shock wave is emitted and with what amplitude depends among other parameters on the properties of the liquid and on the dimensions of the reservoir that contains the liquid. For example, it is known that for liquids with higher viscosity, bubble's oscillations are slower and last longer. In viscous fluids, the dynamics of the collapse is slowed down, reducing the energy of the shock wave. In highly viscous fluids, shock waves are not observed at all.

Similar dependence applies also with regard to the dimensions of the reservoir. In a free liquid, bubble oscillations can be accommodated by displacing the liquid at long distances. However, in a confined environment, a free expansion of the bubble is not possible, and the expansion and contraction of the bubble is slowed down by the added resistance to flow due to the impermeability and the no-slip condition on the reservoir's surface. This process delays the dynamics of bubble's expansion and implosion compared to a free liquid situation. More importantly, because of the slowed down dynamics of the bubble's collapse, shock waves are weaker or do not occur at all.

For small reservoirs, shock waves are therefore weak or are not emitted at all. The cleaning effect of cavity oscillations is therefore limited to rapid liquid streaming and liquid jets, while the potential of much more violent shock waves is not utilized. For example, for dental endodontic cleaning, removing debris from root canal surfaces and eliminating infection consists of adding various chemical solvents into a root canal, and then using a laser irrigation method primarily to enhance the spreading of the chemical irrigant into hard to reach root canal areas. However, without creating shock waves, a sufficiently effective cleaning and disinfecting of small root canals remains elusive when using only water as the irrigating liquid. On the other hand, the use of potentially toxic irrigants is generally not desirable.

EP 3 127 502 A1 addresses this problem by delivering energy to a liquid in a set of a minimum of two individual laser pulses (a prior and a subsequent pulse) that follow temporally each other by an appropriate pulse repetition time (Tₚ ), the pulse repetition time T_{P} being the time period from the beginning of one single pulse p to the beginning of the next, subsequent pulse p. This allows creation of a shock wave by the prior bubble, i.e., the bubble resulting from the prior laser pulse, even in situations when no shock wave is emitted by the bubble when only one laser pulse is delivered to the liquid. This observation is explained by the fact that the liquid pressure exerted on the prior bubble by the expanding subsequent bubble, i.e., the bubble resulting from the subsequent laser pulse, forces the prior bubble to collapse faster, thus facilitating the emission of a shock wave by the prior bubble. An important condition that needs to be fulfilled in order for the above described effect to be observed is that the subsequent bubble starts to develop when the prior bubble is already in its implosion phase.

EP 3 127 502 A1 discloses a feedback system that determines a bubble oscillation intensity and adjustment means to adjust the pulse repetition time T_{P} as a function of the determined bubble oscillation intensity, such that an onset time of a second vapor bubble is within a first contraction phase of a first vapor bubble, when the later has contracted from its maximal volume to a size in the range from about 0.7 to about 0.1 of the maximal volume.

EP 3 127 502 A1 further discloses to use multiple pairs of pulses and to repeatedly vary the time difference between the onset time of the second vapor bubble and the onset time of the related first vapor bubble in a sweeping manner, such that within at least one pair of first and second bubbles, an onset time of a second vapor bubble is within the range indicated in the preceding paragraph.

However, the approaches disclosed in EP 3 127 502 A1 are still not perfect. Therefore, there is a need to improve the known methods, techniques and technologies that can improve the cleaning of small cavities.

### SUMMARY OF THE INVENTION

In an aspect, the above object is at least partly solved by an apparatus for applying pulses of electromagnetic radiation to a cavity filled with a liquid. The apparatus comprises a source for generating a first pulse and a second pulse of electromagnetic radiation. Moreover, the apparatus comprises a control unit adapted to control a time between the first pulse and the second pulse as a function of a diameter D and/or a cross-sectional area of the cavity.

The inventors of the present invention have surprisingly found that it is typically a characteristic dimension of the cavity, e.g., as expressed in a diameter or a corresponding cross-sectional area of the cavity, that has the predominant effect on the required temporal pulse spacing. Hence, they found that the characteristic dimension of the cavity, e.g., as expressed in a diameter or a corresponding cross-sectional area of the cavity, provides a universal parameter that allows simple and effective control of the temporal pulse spacing to ensure generation of a shock wave even in small diameter (or corresponding small cross-sectional area) cavities. A complex feedback system that measures the actual bubble dynamics, i.e. its oscillation intensity, is not needed. Instead, static information about the cavity size is used to effectively control the temporal pulse spacing, which - through a variety of experiments described herein - was identified as a key parameter for pulse spacing control in order to effectively create shock waves also in small cavities. The control as a function of the diameter and/or cross-section also enables to reduce or even eliminate the need for sweeping, as an optimized pulse repetition frequency for the respective cavity diameter may be applied for all pulses.

Notably, the inventors have found out that the geometry of the cavity does not have to be known in a detailed way in order to enhance shock wave generation. Instead, it was found that the optimal time (e.g., pulse repetition time, being the time period from the beginning of one pulse p to the beginning of the next, subsequent pulse p) essentially only depends on an effective or average or mean diameter (or cross-sectional area) of the cavity. In other words, the variation in the bubble oscillation period arising from the variation in the size and shape of the lateral surface of the cavity may adequately be described by a cavity diameter, e.g. minor and/or major diameter, or the mean value thereof. The inventors have used this insight to pre-calibrate the optimum pulse repetition time as a function of the cavity diameter/cross-section and provided a control unit programmed accordingly, resulting in an apparatus that allows substantially improved cleaning of small cavities irrespective of their diameter by taking their diameter into account.

It is noted that the cavity may be a canal (e.g., a root canal (system)), a vessel (e.g., a blood vessel), a urinary tract, a passage, a periodontal pocket, a surgical hole, an opening surface which is to be cleaned or disinfected, and it may generally be any liquid reservoir. In what follows the terms reservoir and/or cavity will be used interchangeably to describe any or all of the applicable liquid reservoirs. For example, the apparatus may particularly be used for application to elongate cavities, whose diameter is small compared to their axial dimension (e.g. having an opening whose diameters are smaller than a depth of the opening). The optical axis of first and second pulses may be approximately parallel to an elongate axis of an elongate cavity.

Particularly, the inventors have found out that for reservoirs whose smallest (lateral) dimension is less than 8 mm, assuming these are filled with water (or a fluid with similar viscosity), shock waves typically do not occur without proper setting of the temporal pulse spacing. The present invention is therefore particularly useful for such smaller reservoirs. It should be appreciated for most typical anatomic liquid reservoirs, such as blood vessels, ureter canals, ocular vitreous cavity, endodontic access openings or periodontal pockets, such small cavity diameters are typically at hand. Nevertheless, based on the present invention, these may be cleaned by shock waves as the temporal pulse spacing can be appropriately set according to the respective cavity diameter/cross-section.

In experiments, the inventors have found that there is an optimal repetition time (Tₚ₋ₒₚₜ) between the pulses in order for the shock wave to occur, and that there should not be too much deviation therefrom. The optimal repetition time (Tₚ₋ₒₚₜ) has been found to be such that the subsequent bubble starts to develop during the second half of the first bubble's oscillation period (T_{B}), e.g., when Tₚ is in a range from about 1.2 x (T_{B}/2) to about 1.95 x (T_{B}/2), preferably in a range from about 1.5 x (T_{B}/2) to about 1.9 x (T_{B}/2), and particularly preferably in a range from about 1.6 x (T_{B}/2) to about 1.9 x (T_{B}/2).

The inventors have found that, in order to achieve the above relations, it is very effective to control the pulse repetition time as a function of a diameter or cross-section of the cavity. This is attributed to the experimental finding that, using the same liquid and the same laser parameters, for the optimal pulse repetition time, it is practically the only quantity that influences the optimal pulse repetition time. In fact, it is generally not necessary to determine the precise geometry of each individual cavity. Rather, it was found that, to significantly improve the setting of the optimal pulse repetition time, it is generally sufficient to determine a diameter and/or cross-sectional area. The temporal pulse spacing can thus be optimized such as to obtain strong secondary shock waves during bubble cavitation oscillations even in confined geometries without much added complexity.

The inventors have used this insight to pre-calibrate the optimum pulse repetition time, for a given liquid and a given set of pulse parameters, as a function of the cavity diameter (or cross-sectional area) and to adapt the control unit accordingly, resulting in an apparatus that allows substantially improved cleaning of small cavities irrespective of their diameter.

Notably, a diameter of a cavity as understood herein may refer to an effective or average diameter at an opening of the cavity or the approximate location of the bubble (e.g., as integrated, or simply defined by half of the sum of the maximum and minimum diameters Dₘₐₓ and Dₘᵢₙ, respectively) that is approximately perpendicular to the optical axis of the first and second pulses. However, also a minor diameter Dₘᵢₙ or a major diameter Dₘₐₓ of such opening can represent an appropriate characteristic dimension and may thus constitute a suitable diameter of a cavity according to the present invention. Their use may particularly be beneficial for procedures where there is a considerable correlation between Dₘᵢₙ and Dₘₐₓ, such as for example exists in endodontic (e.g., root canal) cleaning (with minor and major diameters being the mesiodistal and buccolingual diameters, respectively.). Notably, a diameter may be understood as a diameter, e.g. as measured approximately perpendicular to the optical axis of the first and second pulses.

The cross-sectional area may be understood as effective cross-sectional area at an opening of the cavity or the approximate location of the bubble (e.g. as estimated from a maximum and/or minimum diameter, an integration, etc.), e.g. as the area circumscribed by the cavity walls approximately perpendicular to the optical axis of the first and second pulses.

However, representations of a characteristic dimension of the cavity, other than diameter or cross-sectional area, may also be appropriate when so required by the type of the procedure and of the cavity shape. For example, for procedures where there exists a considerable variation in the cavity's depth (D_{z}), an appropriate characteristic dimension may be represented by D_{z} either alone or in combination with the dimensions of the lateral surface (lateral cross section of the cavity at the location of the bubble and/or the cavity opening approximately perpendicular to the optical axis of the first and second pulse), e.g. diameter, cross-sectional area. In other words, in addition, or alternatively to the control unit being adapted to control the time as a function of a diameter and/or a cross sectional area of the cavity, it is contemplated that it may additionally or alternatively be adapted to control the time as a function of any other characteristic dimension of the cavity.

It is noted that it is also within the scope of the present invention that the optical axis of the second pulse deviates from that of the first optical axis. It is merely decisive that the pulse repetition time is properly set. The terms "approximately perpendicular to the optical axis of the first and second pulses" and "approximately parallel to the optical axis of the first and second pulses" may thus include slight deviations from perfect perpendicularity and/or parallelism, e.g. up to ±40°, ±30°, ±20°, ±10°, or ±5°.

Herein, the terms "liquid" and "fluid" will be used interchangeably; furthermore, the term "cleaning" will be used to describe all or any of the potential mechanical, disinfecting or chemical effects of cavitation oscillations on surrounding environment (e.g., debridement, material removal, irrigation, disinfection, decontamination, e.g. of surfaces of the cavity, cleaning, and/or fragmentation of particles within such cavities). For example, removal of material may refer to removal of material, such as bacteria or debris (e.g., plaque, calculus, dirt, particulate matter, adhesives, biological matter, residue from another cleaning process, dust, stains, etc.) located on surfaces of the liquid reservoir, and/or suspended within the liquid filling the cavity.

Moreover, the terms electromagnetic radiation (e.g., light or laser light) will be used to describe any electromagnetic radiation, where the source of the electromagnetic radiation may be a laser, laser diode, diode, lamp or any other source configured to produce the electromagnetic radiation having the wavelength that is substantially absorbed in the liquid, either in a linear or non-linear regime. A substantial or significant absorption means in the context of the present invention any absorption of the electromagnetic radiation energy to such an extent, that bubbles are generated within the liquid (e.g. as further described below). Said substantial or significant absorption covers in particular the interaction of laser light having a wavelength in a range from above 0.4 µm to 11.0 µm inclusive, including both wavelength in the range from about 1.3 µm to about 11.0 µm being highly absorbed in OH containing liquids, and wavelength in the range from about 0.4 µm to about 1.3 µm being weakly absorbed in OH containing liquids. However, any other suitable radiation and wavelength is covered like IPL (Intense Pulse Light) from flashlamp sources, in particular with wavelength above 1.3 microns or in the UV region when focused, as well as green flashlamp or diode light in blood. A further option within the invention is the use of a radiofrequency (RF) radiation source and its RF radiation. Within the scope of the present invention further wavelengths may be contemplated in particular in combination with liquids having added absorption enhancing additives.

For the purposes of describing the present invention, the conditions under which a laser light is highly absorbed in a liquid is roughly divided into a linear, or thermal regime, and a non-linear regime. A linear absorption regime applies when laser pulse power density in a liquid is not high enough to result in the ionization or in other non-linear interactions with liquid molecules. Typically, lasers with pulse durations in a microsecond or millisecond range (from one microsecond to about 5000 µs), such as flash-lamp pumped free-generation Er:YAG lasers, operate in a linear regime. In this regime, the intensity I of laser light exponentially diminishes with distance x within a liquid according to I ~ exp (-kx), where k (in cm⁻¹) is a linear absorption coefficient of the liquid at the particular laser wavelength. The absorption coefficient k and the corresponding penetration depth, l = 1/k, are strongly wavelength dependent. For example, the penetration depth of the Er:YAG laser wavelength of 2.94 µm in water is approximately 10⁻⁴ cm while the penetration depth of the Nd:YAG laser wavelength of 1.064 µm is 1 cm. According to this definition, laser wavelengths with l > 1000 µm in the linear regime may be defined as "weakly absorbed" wavelengths. For water, and other OH-containing liquids, the applicable range of highly absorbed wavelengths extends from about 1.3 µm, inclusive, to about 11 µm, and the applicable range of weakly absorbed wavelengths extends from about 0.4 µm to 1.3 µm. In another example, when the liquid is blood, the 532-nm wavelength of a frequency doubled Nd:YAG laser, the 585 nm wavelength of the pulsed-dye laser or the 568 nm wavelength of the Krypton laser, are of interest since they are strongly absorbed in blood's oxyhemoglobin, with their k being approximately within 300-500 cm⁻¹ range.

At extremely high laser power densities, on the order of about of 10¹⁰ -10¹¹ W/cm², an "optical breakdown" as a result of the ionization of liquid molecules may occur, leading to an abrupt increase in liquid's absorption. In this, non-linear regime, a high absorption of laser light is observed even for weakly absorbed wavelengths, i.e., for wavelengths which have a long penetration depth p in the linear regime. Non-linear conditions are typically achieved with high pulse power Q-switched laser beams, with pulse durations (tₚ) in a nanosecond range (from one nanosecond to about 100 ns), especially when these beams are focused into a sufficiently small volume of the liquid. But other high pulse power lasers with even shorter pulse durations, in the picosecond and femtosecond range, may be used to generate cavitation in liquids as well.

It is to be appreciated that when an optical path of a weakly absorbed high pulse power laser beam has a focal point located within a liquid, the beam will propagate within the liquid without being appreciably absorbed until it reaches the focal region where the laser power density becomes sufficiently high for non-linear effects to occur. It is only at this point that a bubble formation will occur.

The apparatus for applying pulses of electromagnetic radiation, e.g. including a laser system for generating laser pulses, can be configured to deliver pulses to a liquid in a contact or a non-contact manner. In a contact scenario, the pulses are delivered to the liquid through an exit surface of an optical exit component (e.g., fiber, fiber tip, optical window, lens) which is at least partially submersed into the liquid. The (laser) light's focus is located at the exit surface of the exit component, and the bubble develops in a contact with the exit surface of the submersed optical exit component.

In a non-contact scenario, the optical exit component is configured to be positioned above the surface of the liquid reservoir, with the (laser) pulse energy being directed through air and possibly other transparent materials (such as, for example an eye lens in case of ophthalmic applications) into the liquid reservoir. In a non-contact scenario, the beam is substantially focused to a point located bellow the liquid surface by means of an appropriate focusing device, and the resulting bubble does not develop in a contact with the optical exit component.

It is to be appreciated that the contact manner is more suitable for configurations when (laser) light is absorbed in a linear regime, and the non-contact manner is more suitable for configurations when (laser) light is absorbed in a non-linear regime. However, either of the delivery manners can be used in a linear or a non-linear regime.

Apart from the proper setting of the pulse repetition time, there is a further condition that needs to be fulfilled in order for a shock wave being created also in small cavities: The energy of the subsequent pulse must be delivered at a location nearby the prior bubble but not within the prior bubble. In the opposite case, the energy of the subsequent pulse will not be initially absorbed in the liquid but shall first pass through the prior vapor bubble and will be absorbed at the prior bubble's wall area generally opposite to the direction of the laser beam. This would result in extending the length of the prior bubble in the direction of pulse emission and would therefore shift the bubble's dynamics from the contraction to expansion phase, effectively preventing the formation of a shock wave.

When both pulses are focused to the same spot within the liquid, the second condition can be fulfilled only when the subsequent pulse is emitted when the prior bubble has already moved sufficiently away from its initial position, i.e., from the point in the liquid where energy is being locally absorbed within the liquid. Such movement occurs naturally in contact delivery scenarios where during its contraction phase the bubble separates and moves away from the exit surface of the optical exit component. In one of the embodiments, a highly absorbed wavelength may be delivered into a narrow, tube like reservoir, such as a root canal or a blood vessel, by a submerged fiber or fiber tip. In this configuration, the fluid dynamics has been observed to be such that during its contraction phase the bubble separates from the fiber end and moves away from the fiber. This allows the subsequent bubble to develop at the fiber end separately from the prior bubble, and by its expansion to cause the surrounding liquid to exert pressure on the prior bubble during its contraction.

The bubble may move away from the laser's focal point also in non-contact scenarios, providing that the confined reservoir wall's geometry is asymmetrical with regard to the bubble, and the resulting asymmetrical liquid flow shifts the bubble away from its original expansion position.

In another embodiment, the second condition may be fulfilled by physically moving the fiber to a different position within the liquid during the repetition time of the two laser pulses. In yet another embodiment, it is the laser focal point which may be moved in between the pulses, for example by a scanner.

It is to be appreciated that the invention is not limited to the emission of only two subsequent pulses within a pulse set. A third pulse following a second laser pulse, and fulfilling both conditions, may be delivered resulting in an emission of a shock wave by the previous (second) bubble. Similarly, an n^{th} subsequent laser pulse will result in an emission of a shock wave by the (n-1)^{th} bubble, and so on as further laser pulses are being added to the set of pulses. The more laser pulses are delivered in one pulse set, the higher is the laser-to-shock wave energy conversion, with the energy conversion efficiency being proportional to the ratio (n-1)/n where n is the total number of laser pulses delivered in a pulse set. Additionally, repetitive cavitations and shock wave emissions generate an ever-increasing number of longer persisting gas (e.g., air) micro-bubbles within a liquid. These micro-bubbles compress and expand under the influence of cavitation oscillations and shock waves, and thus improve the overall cleaning efficacy by contributing to the high-speed fluid motion.

In summary, when a pulsed laser beam which is highly absorbed in a liquid, either in a linear or non-linear regime, is delivered to such a liquid, a bubble oscillation sequence develops, typically with a temporal oscillation period (T_{B}) in the range from about 50 µsec to about 1500 µsec. The oscillation is damped and lasts for only a few rebounds due to the bubble's energy being spent for heating, moving and displacing the liquid, and under appropriate conditions, also for emitting shock wave acoustic transients. For the purposes of cleaning it is desirable that as much as possible of the bubble's energy is spent in the emission of violent shock waves during the contraction phases of the bubble's oscillation, and preferably at least during the first bubble's contraction phase when the bubble's energy is still high. However, in spatially small reservoirs or in highly viscous liquids, more energy is wasted for overcoming the friction on the cavity walls and to fight against the resistance of the water which has to be displaced in the small reservoir, and/or for overcoming viscous damping. Consequently, the bubble's maximal volume is reduced, and the bubble's contraction is slowed down, resulting in a lower amplitude shock wave or no shock wave at all, as outlined above. By applying a first and a second pulse with the pulse repetition time controlled according to the present invention, shock waves may be generated in an effective and efficient way, such that also small cavities can be cleaned, irrespective of their diameter.

In an example, the apparatus may further comprise a user interface for receiving information on a diameter of the cavity. Hence, the respective user of the apparatus may input e.g. a value for a diameter of the cavity which he may know or estimate from his general practice for the type of cavity at hand or which he may (e.g. visually) estimate or measure for a specific cavity. Based thereon, the control unit controls the time between the first pulse and the second pulse accordingly. Hence, a very easy-to-operate but at the same time effective cleaning device is provided. It is to be appreciated that the expression "input" is to be understood broadly, describing any means of providing information to the user interface by the user, including but not limited to using typing in, e.g. by a keyboard, a touchpad, a touchscreen, a mouse, and/or a pointing device, also including visual and/or verbal commands.

For example, for dental applications, the user may input a tooth type, a cavity type and/or any other information on a characteristic dimension of the access cavity (for example a minor and/or major diameter, etc.). The control unit may determine, based on the user input (e.g. the tooth type, the cavity type, etc.), further information (e.g. a minor and/or major diameter for the tooth type). Exemplary values for tooth types and further information, e.g., suitable values for minor and/or major diameters associated with the tooth types are shown in Table 1. In some example, (only) two tooth types may be distinguished: a first minor and/or a first major diameter may, e.g., be associated with molar teeth (first type), and a second minor and/or a second major diameter may, e.g., be associated with all other teeth (second type). The further information may be stored in a storage device of the apparatus and/or a remote storage device, e.g. in the form as a look-up table or database. The control unit may, based on the user input and/or the further information automatically adjust the time between the pulses.

In an example, the apparatus may comprise a user interface for receiving information on the cross-sectional area of the cavity. As outlined above regarding a diameter of the cavity, similar benefits may be obtained by using information on the cross-sectional area of the cavity.

In addition or as an alternative to the mentioned user interface, in some examples, the apparatus may further comprise means for determining the diameter and/or cross-sectional area of the cavity. For example, the means for determining may provide, e.g., fully automatically, (information on) a diameter and/or cross-sectional area of the cavity. To this end, the means for determining may comprise a sensor unit. For example, a camera with corresponding image analysis software may be provided, or any other optical and/or acoustic sensor unit for measuring a diameter or cross-sectional area may be provided.

Additionally or alternatively, the means for determining may require operator intervention to determine the (information on) the diameter and/or cross-sectional area. The information may then e.g. be input by the user into the user interface. For example, a (microscope) objective and/or lens may be provided together with a scale (e.g., integrated with the apparatus), such that the operator may simply estimate or read the diameter/cross-section and subsequently enter it into the user interface. In other examples, e.g. a camera is provided such that the corresponding information is automatically determined by the means for determining. In both cases, the information on the diameter/cross-sectional area may be determined in-situ, such that separate measurements with different devices can be avoided.

In some examples, the control unit may be adapted to control the time between the first pulse and the second pulse such that it varies with the inverse root of the diameter of the cavity. As an alternative, also an implementation that lets the time vary as 1/(cross-section of the cavity)^{1/4} or any other mathematical relation that essentially leads to a dependence of the time on the inverse root of a diameter of the cavity is considered to fall within such an implementation. Such control has turned out to be particularly effective. This is attributed to the experimental finding that the bubble oscillation period, and hence the optimal pulse repetition time, approximately varies with the inverse root of the diameter of the respective cavity. This relation is particularly strong for cavity diameters of 8 mm and smaller.

Particularly for the case that the apparatus is to be used for different laser parameters (e.g. laser pulse energies, wavelength, duration, the characteristics of the employed (contact or non-contact) delivery, beam spot size, diameter of fiber tip, beam shape, beam angle, fiber tip shape, etc.) and/or different liquids (e.g. having different viscosity), the control unit may be further adapted to control the time between the first pulse and the second pulse as a function of one or more laser parameters and/or the liquid.

Generally, when the same apparatus is intended to be used for cleaning differently sized cavities, containing different liquids, and with different laser parameters, this poses a challenge since the bubble oscillation time (T_{B}), and consequently the required pulse repetition time (Tₚ₋ₒₚₜ) depends critically on a myriad of parameters, the bubble oscillation time being longer, for example, for higher laser pulse energies and smaller reservoirs, and/or for more viscous liquids.

However, typically all or at least most of these parameters are a-priori known or "controlled". Notably, the inventors of the present invention have found that the set of "controlled" parameters can be reduced to a single parameter that, together with the diameter/cross-section of the cavity can be used to control the temporal pulse spacing.

This is based on the experimental finding that the influence of all controlled parameters (i.e., all parameters except for the cavity dimensions) can be approximately described by a single parameter, the "unconstrained" or free bubble oscillation period (Tₒ) representing the bubble dynamics under the conditions when the cavity dimensions are "infinitely" large, i.e., when the bubble dynamics is not affected by the spatial containment caused by the uncontrolled cavity dimensions. Further, it is our surprising finding that the optimal pulse repetition time (Tₚ₋ₒₚₜ) is determined with sufficient accuracy solely by the known unconstrained bubble oscillation period (T₀) in combination with a characteristic cavity dimension (S), e.g. a diameter or a cross-sectional area of the cavity, characterizing the influence of the uncontrolled cavity dimensions on the damping of the bubble's oscillation.

In an example, the control unit may be adapted to control the time between the first pulse and the second pulse as a function of a predetermined parameter that is specific to at least an energy of the first pulse and/or to the liquid. Hence, the control unit may adapt the time in a particularly easy and efficient way, by simply using a single predetermined parameter (in addition to the information on the cavity diameter/cross-sectional area) corresponding to the respectively used pulse energy and/or liquid. In some examples the predetermined parameter may also be specific to other controlled parameters. Hence, the pulse repetition time may also be adapted to other controlled parameters in a simple manner, such that also for these, an efficient cleaning can be provided.

It is noted that the predetermined parameter may be independent of the geometry of the cavity. That is, it may be a universal parameter that only depends on the controlled parameters.

Moreover, in some examples the predetermined parameter corresponds to an unconstrained oscillation period Tₒ of a bubble that would be generated by the first pulse in an infinitely large cavity filled with the liquid.

In some examples, the control unit is adapted to determine the predetermined parameter by accessing a data storage device of the apparatus and/or a remote data storage device. For example, the predetermined parameter (e.g., unconstrained oscillation period) may be predetermined for each particular liquid and/or pulse energy (and/or further controlled parameters), for example. It may then be made available to the control unit either by storing it on a data storage device of the apparatus and/or a remote data storage device. When the user of the device wants to use different optical power and/or a different liquid (or changes any other controlled parameter), the new optimal pulse repetition time may simply be calculated based on the correspondingly altered predetermined parameter (e.g. the corresponding unconstrained oscillation period and the cavity diameter).

In some examples the predetermined parameter may be stored, as outlined above, in the form of a look up table. For the respectively used controlled parameters, the corresponding predetermined parameter may be read out by the control device, if needed. Based thereon, the temporal pules spacing may then be determined in a simple manner as also outlined above.

Similarly, as described with respect to the information on a diameter and/or cross-sectional area of the cavity, the user may also input the information on the unconstrained oscillation period (e.g. a specific unconstrained oscillation period) corresponding to the particular set of selected controlled parameters into the user interface. For example, the control unit may then control the pulse repetition time accordingly. In other words, the user interface may be adapted to receive information on the unconstrained oscillation period (e.g. a specific unconstrained oscillation period).

The user interface may, additionally or alternatively, be adapted to receive information on one or more controlled parameters and/or the diameter and/or cross-sectional area of the cavity. Additionally or alternatively, the apparatus may be adapted such that the information on one or more controlled parameters and/or the diameter and/or cross-sectional area of the cavity (e.g. as set by a (semi-)automatic mode of the apparatus for a certain mode selected by the user, etc.) are automatically provided to the control unit. The control unit may then determine the predetermined parameter based on the information received by the user interface and/or the information automatically provided.

It is particularly beneficial to unite the influence of all controlled parameters into the predetermined parameter (e.g. the unconstrained oscillation period). For example, if an operator wants to change, e.g. the size of a fiber tip with which the pulses are applied, the corresponding unconstrained oscillation period for the new tip size may be determined, and the corresponding new pulse repetition time may be easily calculated based on this single altered parameter. In some examples, the altered parameter and/or the new pulse repetition time may be displayed by the user interface (e.g., by means of a (touch-)screen, an LCD, TFT and/or LED display, etc.).

In some examples, the control unit may be adapted to control the time between the first pulse and the second pulse such that it is proportional to the predetermined parameter. This control has turned out to be particularly effective. It is attributed to the experimental finding that the bubble oscillation period of the pulses has been found to approximately vary linearly with the predetermined parameter (e.g., the unconstrained oscillation period).

The control unit may be adapted to control the pulse repetition time as a function of information on the diameter and/or cross-sectional area and the predetermined parameter, only.

In some examples, the control unit may be adapted to control the time according to the function K_{D} x Tₒ x D^{-0.5}, wherein K_{D} is selected from the range 2 mm^{0.5} to 4.8 mm^{0.5}, preferably from the range 2.5 mm^{0.5} to 3.8 mm^{0.5}, and more preferably from the range 2.7 mm^{0.5} to 3.8 mm^{0.5} (and wherein D is a diameter of the cavity, and Tₒ is the predetermined "unconstrained oscillation period").

In some examples, the control unit may be adapted to control the time according to the function K_{A} x Tₒ x A^{-0.25}, wherein K_{A} is selected from the range 2 mm^{0.25} to 4.1 mm^{0.25}, preferably from the range 2.5 mm^{0.25} to 3.3 mm^{0.25}, and more preferably from the range 2.7 mm^{0.25} to 3.3 mm^{0.25} (and wherein A is an area of the lateral surface of the cavity, and Tₒ is the predetermined "unconstrained oscillation period").

In some examples, the control unit is adapted to control the pulse repetition time Tₚ such that the subsequent bubble, i.e., the bubble generated by the subsequent laser pulse, starts to expand when the prior bubble has already started to contract, i.e., when Tₚ is in a range from about 1.2 x (T_{B}/2) to about 1.95 x (T_{B}/2), preferably in a range from about 1.5 x (T_{B}/2) to about 1.9 x (T_{B}/2), and expediently in a range from about 1.6 x (T_{B}/2) to about 1.9 x (T_{B}/2). This may be achieved, e.g., by using the aforementioned functional relationship between Tₚ, Tₒ, and D (or correspondingly any characteristic dimension other than D).

Note that in simple embodiments, the controlled parameters may be fixed such that also the unconstrained oscillation period Tₒ may be fixed. The control unit may thus be adapted to adjust the pulse repetition rate Tₚ, as a function of (only) the information on the characteristic dimension S of the cavity and the fixed Tₒ, to at least approximately correspond to the required optimal pulse repetition rate Tₚ₋ₒₚₜ. To this end, the inventive relations, Tₚ₋ₒₚₜ ~ Tₒ x Dₐᵥₑ^{-0.5}, or Tₚ₋ₒₚₜ ~ Tₒ x Aₗₛ^{-0.25} may be used, e.g. using constants K_{D} and K_{A} as outlined herein.

It is noted that, throughout the present disclosure, it is assumed that the first pulse is adapted to generate a first bubble within the liquid, and the second pulse is adapted to generate a second bubble within the liquid. By means of the control of the pulse repetition time by the control unit as described herein, it can be ensured that a shock wave is generated within the liquid, as explained above.

In some examples, the apparatus may further comprise means for providing the liquid to the cavity. This may make the cleaning of the cavity particularly quick and convenient, since all necessary steps may be carried out with a single apparatus.

The control unit may also be adapted to determine an optimal pulse repetition time Tₚ₋ₒₚₜ and to vary pulse repetition times between subsequent pairs of pulses within a range from Tₚ₋ₒₚₜ - δ₁ to Tₚ₋ₒₚₜ + δ₂, wherein δ₁ and δ₂ are selected from the range 10 µs to 300 µs, preferably from 20 µs to 75 µs and more preferably from 25 µs to 75 µs. This may be beneficial since it is ensured that the pulse repetition time is swept within an optimal range, such that the cleaning may be more efficient. This is attributed to the fact that the optimum pulse repetition time (e.g. determined from the "unconstrained bubble oscillation period" and a "diameter" of the cavity) provides an excellent estimation but may still not be 100% accurate. By sweeping the pulse repetition time within a small window around the estimated optimum pulse repetition time, it may be ensured that the true optimum pulse repetition time is achieved. This may be particularly useful for cavities with particularly irregular dimensions. The aspects of the present invention specifically allow to significantly reduce the interval within which the sweeping is to occur such that the efficiency of the cleaning is greatly improved.

In another aspect, a method is provided for applying pulses of electromagnetic radiation to a cavity filled with a liquid. A first pulse and a second pulse of electromagnetic radiation are generated. The time between the first pulse and the second pulse is controlled as a function of a diameter D and/or a cross-sectional area of the cavity.

It is noted that all aspects outlined herein with respect to the apparatus may also be part of the methods described herein, in the form of a corresponding method step, even if not explicitly mentioned.

For example, the method may include the step of controlling the time between the first pulse and the second pulse as a function of a controlled parameter (e.g. a pulse energy or the liquid), or a predetermined parameter that is specific to at least an energy of the first pulse and to the liquid.

For following the above mentioned inventive findings, the pulses as they are known in the prior art may be replaced by pulse sets according to the present invention whose temporal spacing or pulse repetition time (i.e., the time from the beginning of a pulse until the beginning of the subsequent pulse) is controlled accordingly. The individual pulses may be combined to pulse sets consisting of a minimum of two and maximally 20 individual pulses, with the intra-set pulse repetition times typically in the range from 50 µsec to 900 µsec, and the pulse sets being temporally separated from each other typically by at least 10 ms.

A further aspect is the use of the laser pulses as described herein for application to a cavity filled with a liquid, and in particular for cleaning the cavity.

The proposed laser system and method may be used for any kind of human or animal cavity (e.g. body or anatomical cavities), or any non-human or non-animal cavity, e.g. industrial or machinery cavities.

According to further examples, the apparatus may be provided as a cleaning system that is configured for cleaning of cavities filled with a liquid. The cavities may have lateral surface characterized by a minor inner diameter and/or major inner diameter (Dₘᵢₙ, Dₘₐₓ), that vary from cavity to cavity. The cleaning system may comprise an electromagnetic radiation system, a control unit and, optionally, the liquid. The electromagnetic radiation system may comprise a radiation source for generating a radiation beam and an optical delivery system for the radiation beam. The delivery system may include a handpiece with an exit component, wherein the exit component may be configured to be inserted into the cavity with an insertion depth (h), wherein the handpiece and the exit component are configured to irradiate the liquid within the cavity with the radiation beam. The wavelength of the radiation beam may be chosen for significant absorption of the radiation beam in the liquid. The electromagnetic radiation system is adapted to be operated in pulsed operation with at least one pulse set containing at least two individual pulses (p) having each an individual pulse energy, wherein within the pulse set a first pulse (pₐ) of the pulses (p), having a pulse duration (tₚ) and pulse energy (E_{L}), is followed by a second pulse (p_{b}) of the pulses (p) with a pulse repetition time (Tₚ). The electromagnetic radiation system is adapted to generate a first vapor bubble within the liquid by means of the corresponding first pulse (pₐ) and to generate a second vapor bubble within the liquid by means of the corresponding second pulse (p_{b}). The controlled parameters of the cleaning system and the cavity may be characterized by the unconstrained oscillation period Tₒ of the first vapor bubble for cavities with infinitely large minor and major diameter, wherein the control unit (22) is adapted to adjust for each cavity the pulse repetition time (Tₚ) to the optimal pulse repetition time (Tₚ₋ₒₚₜ) depending on the unconstrained oscillation period Tₒ of the first vapor bubble and on the size of the lateral surface, such that the interaction between the first vapor bubble and the second vapor bubble generates a shock wave within the liquid.

The control unit may be adapted to adjust the pulse repetition time (Tₚ) to be varied or "swept" in discreet positive or negative steps Δ from an initial pulse period Tₚₒ to a final pulse period Tₚₘ , preferably +- across a range from Tₚₒ = Tₚ₋ₒₚₜ - δ₁ to Tₚₘ = Tₚ₋ₒₚₜ + δ₂ (or from Tₚₒ = Tₚ₋ₒₚₜ + δ ₂ to Tₚₘ = Tₚ₋ₒₚₜ - δ ₁ in the case of a negative Δ), where δ ₁ and δ₂ are each preferably in a range from 10 to 300 µsec, even more preferably in a range from 20 to 75 µsec, and expediently in a range from 25 to 75 µsec.

The control unit may be adapted to calculate the optimal pulse period (Tₚ₋ₒₚₜ) from the unconstrained bubble oscillation period (T₀) and an average diameter (Dₐᵥₑ) of the lateral surface (Dₐᵥₑ) using Tₚ₋ₒₚₜ = F_{S} x Tₒ x Cₐᵥₑ x Dₐᵥₑ^{-0.5} whereas the average diameter coefficient (Cₐᵥₑ) is equal to Cₐᵥₑ = 3.74 mm^{0.5} and whereas the shock wave enhancing factor (F_{S}) is in a range from about 0.6 to about 1.2, preferably in a range from about 0.75 to 0.95, and expediently in a range from about 0.8 to about 0.95.

Similarly, according to an aspect, a method is provided for cleaning a cavity, e.g. a dental root canal, filled with liquid, such as water or another irrigant. The method comprises the following steps:
- providing a laser system comprising a laser source for generating a laser beam, an optical delivery system, optionally a handpiece including an exit component, and adjusting means, wherein the handpiece and its exit component may be configured to irrigate the anatomical cavity in a contact manner, wherein a wavelength of the laser beam may be in a range from above 1.3 µm to 11.0 µm inclusive, wherein the laser system is adapted to be operated in pulsed operation with pulse sets containing at least two and maximally twenty individual pulses (p) of a temporally limited pulse duration (tₚ), wherein the repetition time (tₛ) between the pulse sets may be ≥ 10 ms, and wherein the individual pulses (p) follow one another, optionally with a fixed pulse repetition time Tₚ, wherein the control unit is adapted to adjust the pulse repetition time Tₚ as a function of the unconstrained oscillation period Tₒ of the first vapor bubble and/or of the cavity minor inner diameter (Dₘᵢₙ) and/or major inner diameter (Dₘₐₓ), e.g. as outlined herein;

- applying said pulsed laser beam to the liquid disposed within the anatomical cavity to form at least one prior vapor bubble and a at least one subsequent vapor bubble in the liquid, in order to achieve at least one shock wave emitted by a prior vapor bubble;
- performing the until desired cleaning is achieved, or until the temperature rise within the anatomical cavity exceeds 3.5 degrees Celsius, whichever occurs first.

Alternatively, a sweep configuration may be used instead of a fixed pulse repetition time (Tₚ), wherein Tₚ is being swept in a range from Tₚ₋ₒₚₜ - 50 µs to Tₚ₋ₒₚₜ + 50µs.

More generally, various shortcomings of prior art medical and biomedical devices and methods ("medical" is understood as including "dental" techniques, for example, endodontic techniques, and other "medical" techniques) can be addressed by utilizing an apparatus or other exemplary system configured in accordance with principles of the present disclosure. Outside of the medical field, control of bacteria or other undesirable matter, such as dirt, particulate matter, adhesives, biological matter, residues, dust and stains, in various systems is also important. Further, cleaning and removal of various materials from surfaces and openings may be required for aesthetic or restoration reasons.

### BRIEF DESCRIPTION OF THE DRAWINGS

Embodiments of the invention will be explained in the following with the aid of the drawings in more detail. With reference to the following description, appended claims, and accompanying drawings:
- Fig. 1: illustrates an exemplary inventive laser system with both an optical fiber laser delivery system and an articulated arm laser delivery system;
- Fig. 2a: illustrates an exemplary handpiece fed by an articulated arm in contact operational mode;
- Fig. 2b: illustrates an exemplary handpiece fed by a delivery fiber in contact operational mode;
- Fig. 3a: illustrates an exemplary handpiece fed by an articulated arm in non-contact operational mode;
- Fig. 3b: illustrates an exemplary handpiece fed by a delivery fiber in non-contact operational mode;
- Fig. 4a: illustrates an exemplary optical exit component of a handpiece fed by an articulated arm, having a flat tip geometry, and showing the resultant laser beam path;
- Fig. 4b: illustrates an exemplary optical exit component of a handpiece fed by an articulated arm, having a conical tip geometry, and showing the resultant laser beam path;
- Fig. 5a: illustrates an exemplary vapor bubble in generally spherical form;
- Fig. 5b: illustrates an exemplary vapor bubble in generally elongate form;
- Fig. 6: illustrates an exemplary vapor bubble oscillation sequence under influence of one short laser pulse;
- Fig. 7a: illustrates an exemplary dependence of a single laser pulse vapor bubble oscillation period on the diameter of a confined cylindrical liquid;
- Fig. 7b: illustrates an exemplary dependence according to Fig 7a of the ratio between the single laser pulse vapor bubble oscillation period in a confined cylindrical liquid, and the single laser pulse vapor bubble oscillation period in a large reservoir, on the diameter of the cylindrical cavity;
- Fig. 7c: illustrates an exemplary dependence according to Fig 7a of the ratio between the single laser pulse vapor bubble oscillation period in a confined cylindrical liquid, and the single laser pulse vapor bubble oscillation period in a large reservoir, on the lateral surface of the cylindrical cavity;
- Fig. 8a: illustrates an exemplary collapse and shock wave emission of a vapor bubble under the influence of an expanding subsequent bubble in confined reservoir, according to the present invention;
- Fig. 8b: illustrates an exemplary sequence of laser pulses, and exemplary development of vapor bubbles and emission of a shock wave, according to the present invention;
- Fig. 9a: illustrates an exemplary arbitrarily shaped cavity being cleaned by an exemplary handpiece fed by a delivery fiber;
- Fig. 9b: represents an enlarged diagrammatic illustration of a lateral surface of an arbitrarily shaped cavity according to Fig. 9a.
- Fig. 10a: illustrates an exemplary endodontic access opening being cleaned by an exemplary handpiece fed by a delivery fiber;
- Fig. 10b: illustrates an exemplary endodontic access opening according to Fig. 10a;
- Fig. 11a: illustrates an exemplary dependence of a single laser pulse vapor bubble oscillation period on the average diameters of endodontic access cavities;
- Fig. 11b: illustrates an exemplary dependence according to Fig 11a of the ratio between the single laser pulse vapor bubble oscillation period in a confined and unconfined endodontic access cavity, on the average diameter of the endodontic access cavity.
- Fig. 11c: illustrates an exemplary dependence according to Fig 11a of the ratio between the single laser pulse vapor bubble oscillation period in a confined and unconfined endodontic access cavity, on the area of the lateral surface of the endodontic access cavity.
- Fig. 12: represents a diagrammatic illustration of the temporal course of pulse sets in accordance with various embodiments of the invention;
- Fig. 13: represents an enlarged diagrammatic illustration of a detail of a pulse set according to Fig. 12 with the temporal course of individual pulses with sweeping pulse repetition rates from pulse to pulse within one pulse set;
- Fig. 14: represents an enlarged diagrammatic illustration of a detail of the temporal course of pulse sets according to Fig. 12 with the temporal course of individual pulses with sweeping pulse repetition rates from pulses to pulse set; and
- Fig. 15: represents an enlarged diagrammatic illustration of a detail of an alternative pulse set according to Fig. 12 with the temporal course of individual pulses with sweeping pulse energy from pulse to pulse within one pulse set.

### DETAILED DESCRIPTION OF PRESENTLY PREFERRED EMBODIMENTS

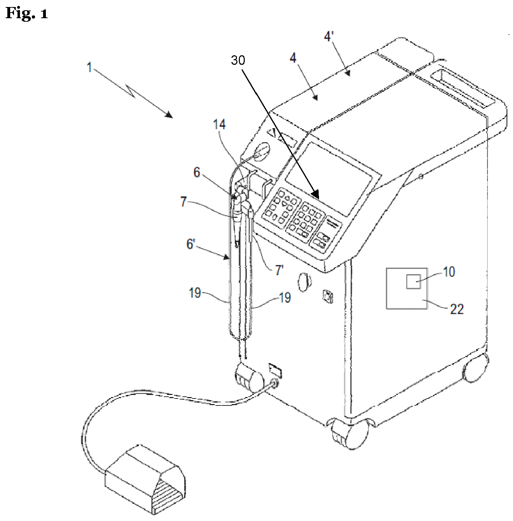

With reference now to Fig. 1, in various embodiments, an electromagnetic radiation system comprising a radiation source for generating a radiation beam is shown. In the following, both the inventive electromagnetic radiation system and an inventive method of operating said electromagnetic radiation system are described.

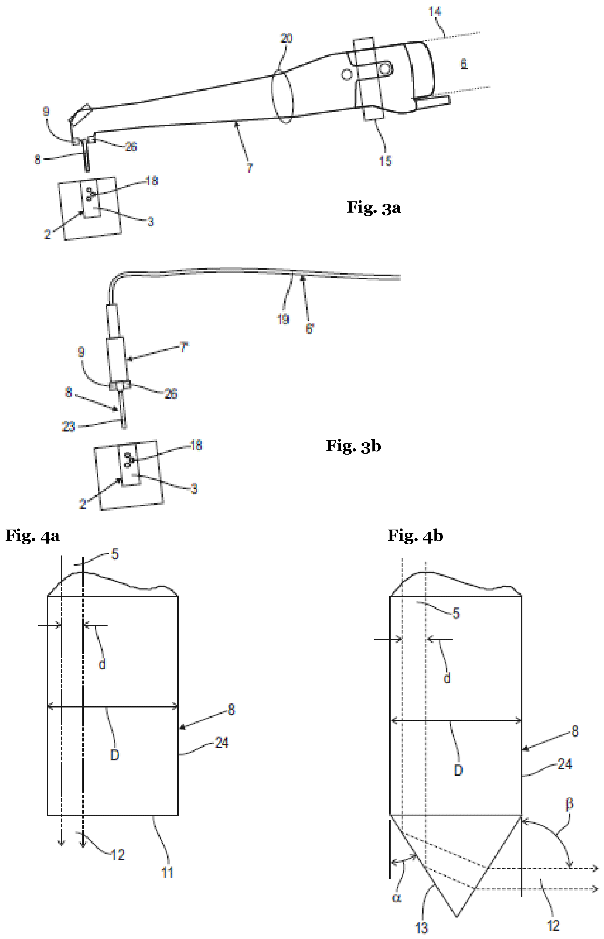

In the shown preferred embodiment, the apparatus is implemented as electromagnetic radiation system, and more particularly, laser system 1. The source for generating pulses is implemented as laser source 4, generating a radiation beam, more particularly a laser beam 5, e.g. including laser pulses. Laser system 1 comprises at least one laser source 4 for generating at least one laser beam 5 (cf. Figs. 4a and 4b for more detail), and at least one optical delivery system 6 for the laser beam(s) 5.

Laser system 1 further comprises a schematically indicated control unit 22 for controlling laser beam 5 parameters, wherein control unit 22 includes again schematically indicated adjusting means 10 for adjusting the laser beam 5 parameters as described herein, particularly for controlling the pulse repetition time.

The control unit may be implemented as a computer-related entity, either hardware, firmware, a combination of hardware and software and/or firmware, software, or software in execution, e.g. as a computer. The various functions of the control unit may be implemented in hardware, software, firmware, or any combination thereof. If implemented in software, the functions can be stored on or transmitted over as one or more instructions or code on a data store. A data store can be any available media that can be accessed by a computer. By way of example, and not limitation, such computer-readable media can comprise RAM, ROM, EEPROM, CD-ROM or other optical disk storage, magnetic disk storage or other magnetic storage devices, or any other medium that can be used to carry or store desired program code in the form of instructions or data structures and that can be accessed by a computer. Also, any connection is properly termed a computer-readable medium. For example, if the software is transmitted from a website, server, or other remote source using a coaxial cable, fiber optic cable, twisted pair, or digital subscriber line (DSL), or wireless technologies such as infrared, radio, and microwave, then the coaxial cable, fiber optic cable, twisted pair, or DSL are included in the definition of medium.

Optical delivery system 6 preferably includes an articulated arm 14 and/or a handpiece 7, wherein the laser beam 5 is transmitted, relayed, delivered, and/or guided from the laser source 4 through the articulated arm 14 and the handpiece 7 to a target. The articulated arm 14 might preferably be an Optoflex^{®} brand articulated arm available from Fotona, d.o.o. (Slovenia, EU). In the shown preferred embodiment, a second laser source 4' and a second optical delivery system 6' with a second handpiece 7' is provided, wherein instead of the articulated arm a flexible elongated delivery fiber 19 for guiding the laser beam 5' is incorporated. Despite both laser sources 4, 4' and delivery systems 6, 6' being shown in combination, one of both in the alternative may be provided and used within the scope of the present invention.

Moreover, laser system 1 may be configured with any appropriate components and/or elements configured to facilitate controlled application of laser energy, for example, in order to create vapor bubbles in a liquid 3 within a cavity 2 for cleaning (including debridement, material removal, irrigation, disinfection, and/or decontamination of said cavity 2 and/or fragmenting particles within such cavities), as shown and described herein.

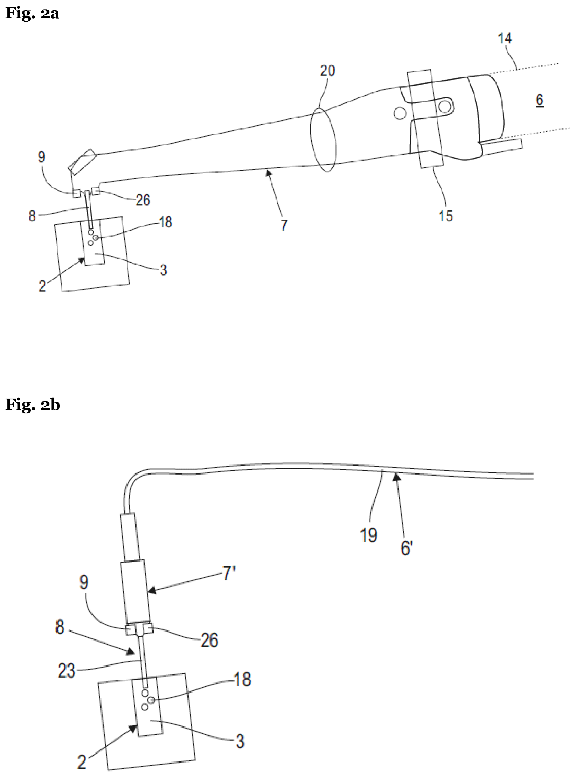

With reference now to Figs. 2 and 3, it is to be understood that the cleaning according to the invention is intended for a cavity 2 (Figs. 2, 3) filled with a liquid 3. In case of medical or dental applications, cavity 2 may be filled spontaneously with blood or other bodily fluids by the body itself. Alternatively, the cavity may be filled with water, or other liquids such as disinfecting solutions, by the operator and/or the apparatus. In the embodiments of Figs. 2 and 3, the apparatus may be designed to include a liquid delivery system 26 configured to fill the volume of the cavity with the liquid. Preferably, said liquid 3 is an OH-containing liquid, for example a liquid with its major portion being water. In other examples, the liquid 3 may include abrasive materials or medication, such as antibiotics, steroids, anesthetics, anti-inflammatory medication, antiseptics, disinfectants, adrenaline, epinephrine, astringents, vitamins, herbs, and minerals. Furthermore, the liquid 3 may contain an additive enhancing the absorption of introduced electromagnetic radiation.

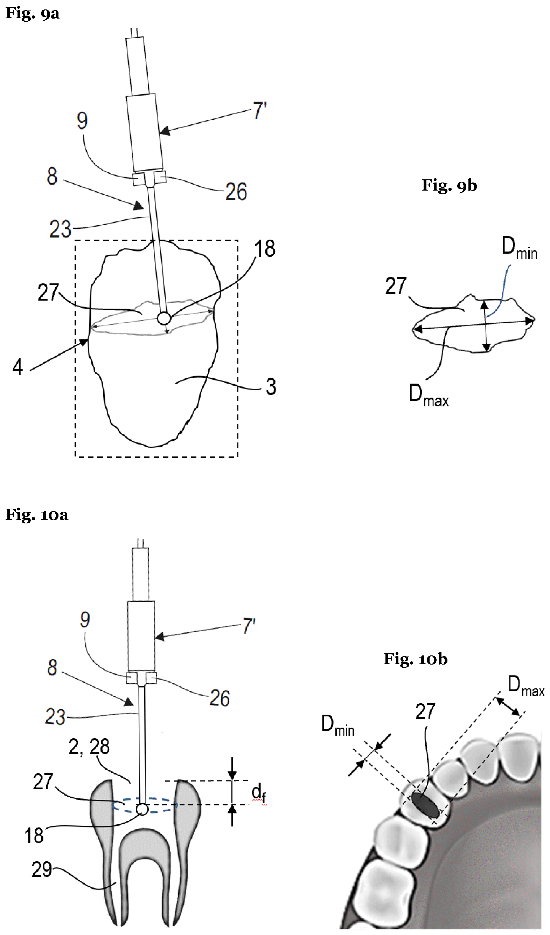

The laser source 4 may be a pulsed laser. The laser source 1 may be solid state laser source and configured with a pulse duration of less than 500 µs. The laser pulse duration is defined as the time between the onset of the laser pulse, and the time when 50% of the total pulse energy has been delivered to the liquid. The pulse duration may be fixed; alternatively, the pulse duration may be variable and/or adjustable. The pulse energy may be fixed; alternatively, the pulse energy may be variable and/or adjustable. The wavelength of the laser beam 5 may be in a range from (above) 0.4 µm to 11.0 µm inclusive. As illustrated in Figs. 9, 13, 14 and 15, the laser system 1 may be adapted to be operated in pulsed operation with pulse sets containing at least two and maximally twenty individual pulses p of a temporally limited pulse duration tₚ, wherein a temporal separation Tₛ between the pulse sets typically is ≥ 10 ms, and wherein the individual pulses p follow one another with a pulse repetition time Tₚ within a range of 50 µs, inclusive, to 1000 µs, inclusive.

The laser source 4, 4' may desirably be configured to generate coherent laser light having a wavelength such that the laser beam 5 is highly absorbed in the liquid 3, wherein the laser pulse duration is in the range of ≥ 1 µs and < 500µs, and preferably of ≥ 10 µs and < 100µs.

Preferably, the laser source 4, 4' is one of an Er:YAG solid state laser source having a wavelength of 2940 nm, an Er:YSGG solid state laser source having a wavelength of 2790 nm, an Er:Cr:YSGG solid state laser source having a wavelength in a range of 2700 to 2800 nm, an Er:YAlO3 solid state laser having a wavelength of 2690 nm, a Ho:YAG solid state laser having a wavelength of 2100 nm, a CO₂ or CO gas laser source having a wavelength of 9000 nm to 10600 nm, all of them providing a laser beam 5 highly absorbed in water and other OH-containing liquids.

In particular, the laser source 4 and/or laser source 4' may be an Er:YAG laser having a wavelength of 2940 nm, wherein the laser pulse energy is in a range from 1 mJ to 100 mJ, preferably from 1 mJ to 40 mJ, and more preferably within a range from 5.0 mJ to 20.0 mJ. This type of laser source may be combined with an exit component 8 that is cylindrical, having a diameter between 200 µm and 1000 µm, wherein the conical output surface 13 has a conical half angle α being in the range from 16° to 38°, preferably from 34° to 38°, wherein the temporal separation T_{S} between pulse sets 21 is < 0.5 s, and wherein the cumulative delivered energy during a cleaning session is below 150 J.

Additionally or alternatively, laser source 4 and/or laser source 4' is configured to generate coherent light having a wavelength highly absorbed in OH-containing liquids, e.g., by means of one of an Er:YAG laser source having a wavelength of 2940 nm, an Er:YSGG laser source having a wavelength of 2790 nm, an Er:Cr:YSGG laser source having a wavelength of 2780 nm or 2790 nm, or a CO₂ laser source having a wavelength of about 9300 to about 10600 nm. The laser pulse energy may be in a range from 1 mJ to 500 mJ.

Other examples of laser sources 4,4' with a laser wavelength highly absorbed in water and other liquids include quadrupled Nd:YAG laser which generates light having a wavelength of 266 nm; an ArF excimer laser which generates light having a wavelength of 193 nm, an XeCl excimer laser which generates light having a wavelength of 308 nm, and a KrF excimer laser which generates light having a wavelength of 248 nm.

In another embodiment, laser source 4 and/or laser source 4' may be one of a frequency doubled Nd:YAG laser source having a wavelength of 532 nm, a dye laser source having a wavelength of 585 nm, or a Krypton laser source having a wavelength of 568 nm, all of them providing a laser beam 5 highly absorbed in oxyhemoglobin within blood vessels.

Alternatively, the laser source 4, 4' may be configured to generate coherent laser light having a wavelength such that the laser beam 5 is weakly absorbed in the liquid 3, wherein the laser pulse duration is in the range of ≥ 1 fs and < 100 ns, and preferably of ≥ 1 ns and < 25 ns. To this end, the laser source 4 and/or the laser source 4' may be one of a Q-switched Nd:YAG laser source having a wavelength of 1064 nm, a Q-switched ruby laser source having a wavelength of 690 nm, or an alexandrite laser source having a wavelength of 755 nm, including laser sources 4, 4' with frequency doubled wavelengths of these laser sources 4, 4', all of them providing a laser beam 5 weakly absorbed in water and other OH-containing liquids. For such weakly absorbed wavelength the pulse energy of one individual laser pulse p is in the range from 0.05 mJ to 1000 mJ, preferably in the range from 0.5 to 200 mJ, and in particular from 1 mJ to 20 mJ.

Moreover, any other suitable laser source 4, 4' may be utilized, as desired. In certain embodiments, the laser source may be installed directly into the handpiece 7, 7', and no further laser light delivery system 6, 6' such as the articulated arm 14 or elongated delivery fiber 19 is required. Additionally, such handpiece may not be intended to be held in hand but may be built into a table-top or similar device as is the case with laser photo-disruptors for ocular surgery.

Laser system 1 comprises a user interface 30. User interface 30 comprises a screen and a plurality of keys and/or buttons.

The handpiece 7, 7' includes an exit component 8, through which the laser beam 5 exits the delivery system 6, 6' for entering the liquid 3, as shown in Figs. 2a, 2b, 3a and 3b. The handpiece 7, 7', and in particular its exit component 8 may be configured to deliver the laser light to the liquid 3 in a contact, and/or non-contact manner. Turning now to Fig. 2a, when the handpiece 7 is configured for a contact delivery, the laser light is from the said "contact" handpiece 7 directed into a "contact" exit component 8 which is configured to be at least partially immersed into the liquid 3 within the anatomical cavity 2 in such a manner that the laser light exits the exit component 8 within the liquid 3, at a depth of at least 1mm, and preferably of at least 3 mm, in order to generate vapor bubbles 18 within the liquid 3, and in order for the laser generated vapor bubble(s) 18 to interact with the liquid-to-cavity surface. In various embodiments, the contact exit component 8 may comprise or consist of an optical fiber tip as shown in and described along with Fig. 2b and Fig. 3b or a larger diameter exit tip 24 as shown in and described along with Figs. 4a and 4b. In certain embodiments (Fig. 2a), the handpiece 7 together with a contact exit component 8 comprises a H14 tipped laser handpiece model available from Fotona, d.d. (Slovenia, EU). And in certain embodiments, an ending of an elongated delivery fiber 19 of the laser light delivery system 6 may be immersed into the liquid 3, thus serving the function of a contact exit component 8 (Fig. 2b).

For the "contact" scenario as shown in Figs. 2a and 2b one of the above described highly absorbed or weakly absorbed wavelengths including all other above described parameters is preferably used, thereby generating at least two vapor bubbles 8 within the liquid 3.

In one of the embodiments of our invention, the laser system 1 comprises a sensor system 9 to determine a characterizing dimension of the cavity, e.g. of its lateral surface 27. For example, the sensor may determine information on a diameter and/or a cross-sectional area of the cavity and provide it to the control unit. The sensor system 9 preferably comprises an optical and/or an acoustical measurement sensor for sensing the lateral surface size.

Furthermore, the laser system 1 comprises control unit 22 for controlling the pulse repetition time Tₚ to achieve at least approximately that the subsequent bubble 18', i.e., the bubble 18' generated by the subsequent laser pulse p_{b}, starts to expand when the volume of the prior bubble 18 has already contracted to the desired size as described above. This control may be implemented by control unit 22, e.g. via its adjusting means 10 adapted to adjust the repetition time of pulses emitted by laser source 4 and/or 4'. For example, the control unit may determine a specific repetition time, and trigger the adjusting means 10 to control the laser source accordingly. For example, the adjusting means may be an actuator of the laser source or it may simply an electronic control input of the laser source that alters the pulse repetition time according to steps as known in the art. The control unit may thus automatically ensure that pulses are delivered at the appropriate Tₚ₋ₒₚₜ depending on the cavity dimension (e.g. diameter, cross-sectional area) and/or one or more controlled parameters. However, the laser pulse repetition time Tₚ might also be manually adjusted by the user, e.g. to be approximately equal to Tₚ₋ₒₚₜ, e.g., corresponding to the cavity dimension and/or one or more controlled parameters.

When the handpiece 7, 7', and its exit component 8 are configured for a non-contact delivery (Figs. 3a, 3b), the "non-contact" exit component 8 of the said "non-contact" handpiece 7 is configured to be positioned above the surface of the liquid 3 reservoir, with the laser energy being directed through air and possible other transparent materials (such as, for example an eye lens in case of ophthalmic applications) into the liquid 3 reservoir. In certain embodiments, a laser source 4 with a highly absorbed wavelength might be used as described above, and the exiting laser beam 5 is substantially focused onto the liquid 3 surface. In the shown "non-contact" scenario, however, preferably a laser source 4 with a weakly absorbed wavelength is used as described above, and the beam is substantially focused to a point located below the liquid surface by means of an appropriate focusing device, e.g. a lens system 20. The weak absorption allows the laser beam 5 to penetrate the liquid 3 until a certain penetration depth where the focal point is located. In the area of the focal point the laser energy concentration is high enough to generate the desired at least one vapor bubble 18, despite the weak absorption. In certain embodiments (Fig. 3a), non-contact handpiece 7, together with a non-contact exit component 8 comprises an H02 tip-less handpiece model available from Fotona, d.d. (Slovenia, EU). And in certain embodiments, an exit component 8 consists of an ending of an elongated laser light delivery fiber 19, which is positioned above the surface of a liquid 3 reservoir (Fig. 3b). Of course, a separate exit component 8 as described along with Fig. 2a might be used for the embodiments of Figs. 2b, 3a and 3b as well. In yet other embodiments, the exit component 8 may represent a focusing optical system consisting of one or more lenses, such as is the case in ocular surgery photo-disruption procedures.

Moreover, handpiece 7 may comprise any suitable components or elements configured for targeted and/or controllable delivery of laser energy to a liquid 3. Preferably, the laser system 1 comprises a scanner 15 as schematically indicated in Figs. 2a, 3a, which allows scanning of the exit component 8 cross section with the laser beam 5, as shown in Figs. 4a, 4b.

Turning now to Figs. 4a and 4b, in various embodiments the exit component 8, preferably but not coercively configured for contact delivery, may consist of an exit tip 24 (Figs. 4a, 4b) or any other optical element, which extends along a longitudinal axis and is made of a material which is transparent to the laser beam. The exit component 8 preferably has a generally circular cross section, which leads to a generally cylindrical shape. However, any other suitable cross section may be chosen. The exit tip 24 may be of a variety of different shapes (e.g., flat, pointed, conical, angled, beveled, double-beveled), sizes, designs (e.g., side-firing, forward-firing) and materials (e.g. glass, sapphire, quartz, hollow waveguide, liquid core, quartz silica, germanium oxide). Further, the exit component 8 may comprise mirrors, lenses, and other optical components.

In one preferred embodiment the exit tip 24 of the exit component 8 has a flat output surface 11 (Fig. 4a). The exit tip 24 of the exit component 8 has a diameter d_{C}, while the laser beam 5 has a diameter d_{L}. The diameter d_{C} of the exit component 8 can be equal to the diameter of the elongated delivery fiber 19 and in particular equal to the diameter d_{L} of the laser beam 5. In the embodiment of Fig. 4a, where the exit component 8 is in the form of a larger diameter exit tip 24, the diameter d_{C} of the exit component 8 is substantially greater than the diameter d_{L} of the laser beam 5. In connection with the a.m. scanner 15 a certain scanning pattern on the flat output surface 11 can be achieved, thereby generating exiting beam portions 12 and as a result vapor bubbles 18 at corresponding locations within the liquid 3 (Figs. 2a, 3a), as may be desired.

In another embodiment as shown in Fig. 4b, the exit component 8, again in the form of a larger diameter exit tip 24, has a pointed end with conically shaped output surface 13 being disposed around the longitudinal axis and having an apex facing away from the incoming beam section, wherein the conically shaped output surface 13 has a half opening angle α being adapted to provide partial or preferably total reflection of the incoming beam section into a reflected beam section within the exit component 8 and to provide refraction of the reflected beam section into an exiting beam portion 12 emerging from the exit component 8 through the conically shaped output surface 13 in approximately radial direction relative to the longitudinal axis. In various embodiments, the angle β is expediently in the range 60° ≤ β ≤ 120°, and preferably about 90°.

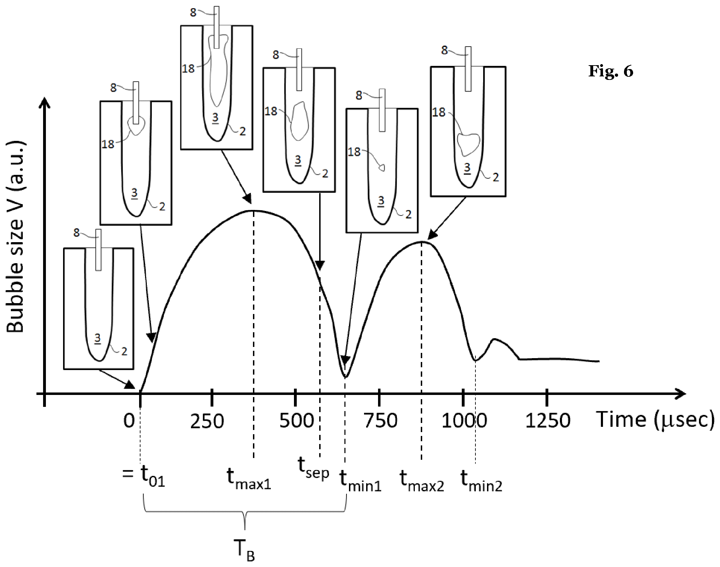

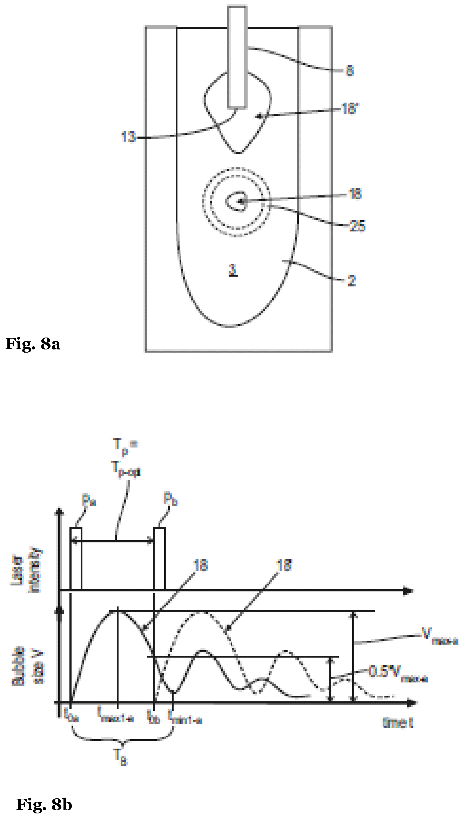

Typically, when fiber tips with output surface 13 are used, the laser beam 5 extends substantially across the whole output surface 13. This will result in a circumferentially spread exiting beam portion 12. In certain embodiments, however, as shown in Fig. 4b, the exit component 8 may have a diameter d_{C} substantially larger than the diameter d_{L} of the laser beam 5, providing space for the laser beam to be scanned over the exit component's conical output surface 13. In such embodiments, the exit component 8 base is preferably of a cylindrical shape. However, any other suitable 3D shape, such as a cube, cuboid, hexagonal prism or a cone, can be used. Scanning the conical output surface 13 with the incoming laser beam 5 allows for generation of multiple exiting beam portions 12 and corresponding vapor bubbles located circumferentially around the exit component 8. Since more than one laser pulse p, i.e. a synchronized train of pulses p (Figs. 8a, 9, 13, 14 and 15) needs to be delivered to the same spot, one could deliver one pulse p exiting beam portion 12 to a related vapor bubble 18 spot, then move to the next vapor bubble 18 spot on the circumference, and so on, and then return to the same initial vapor bubble 18 spot just in time for the next pulse p within the pulse train. This would enable faster procedures since the laser repetition rate would not be limited by the bubble oscillation period T_{B} = (tₘᵢₙ₁-tₒ₁) (Fig. 6) but only by the maximum repetition rate of the laser system 1. In some examples, the apparatus may thus comprise a scanner that directs subsequent pulses to different positions but revisits each position at least once to deliver at least a second pulse there, wherein the pulse repetition rate at each position is controlled by the control unit as described.

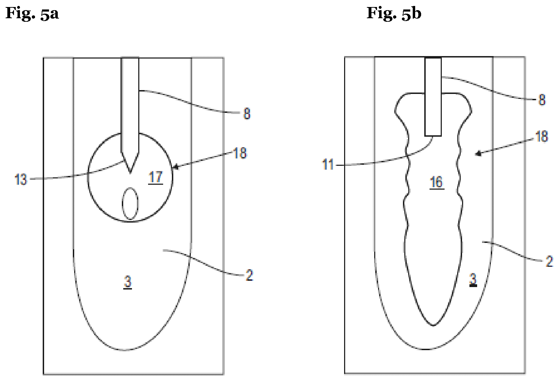

With reference now to Figs. 4a, 4b, in accordance with various embodiments, when laser energy is delivered into a highly absorbing liquid 3 through an exit component 8 having a flat output surface 11 (Fig. 4a), that is immersed into the liquid 3, the above described vapor bubble 18 turns into a channel-like, extended or elongate vapor bubble 16, as schematically indicated in Fig. 5b. A channel-like bubble formation may be generated also when laser energy is delivered to a tubular cavity. On the other hand, when highly absorbed laser energy is delivered into a liquid 3 through an immersed conical output surface 13, or a flat output surface 11 of sufficient small diameter compared to the beam diameter d, or when weakly absorbed laser energy is delivered in "non-contact" mode and focused within the liquid 3 as described above, a generally spherical vapor bubble 18 develops, as schematically indicated in Fig. 5a. It is to be appreciated, however, that in reservoirs with small dimensions, the bubble's shape will be influenced more by the reservoir's geometry, and less by the fiber tip's output surface.

It is also to be appreciated that with shock waves generated according to present invention, conically shaped tips may get more quickly damaged during the violent shock wave emission, and therefore it may be advantageous to use flat surface fiber tips with the present invention.

Moreover, it is to be appreciated, that when in certain embodiments a weakly absorbed laser beam is delivered to a liquid 3 in a non-contact manner, and the beam's focus is located within the liquid 3, and away from the liquid surface, no bubble gets formed at or near the liquid's surface. Instead, the beam gets transmitted deeper into the liquid, and providing that the pulse duration is sufficiently short (≤ 100 ns), and the power density at the focal point within the liquid is sufficiently high, a bubble 18 is generated only when the laser beam 5 reaches its focal point deeper within the liquid 3.

Turning now to Fig. 6, in various embodiments, the system, apparatus and method described herein utilizes an improved scientific understanding of the interaction of pulsed laser light with a highly absorbing liquid 3. When one pulse p of a pulsed laser beam 5 is delivered to such a liquid 3 at an onset time tₒ₁, a bubble oscillation sequence develops. In the 1st phase of the bubble oscillation sequence (from time tₒ₁ to time tₘₐₓ₁), laser energy deposition into the liquid 3 via absorption causes superheating of the liquid 3, and boiling induces a vapor bubble 18. The vapor bubble 18 expands rapidly, and thereafter reaches its maximum size at tₘₐₓ₁, when the internal pressure matches the pressure in the surrounding liquid 3.

In the 2^{nd} phase (from time tₘₐₓ₁ to time tₘᵢₙ₁), the internal pressure is lower than the pressure in the surrounding liquid 3, and this difference in pressures forces the vapor bubble 18 to collapse.

When the vapor bubble 18 collapse completes at time tₘᵢₙ₁, a rebound occurs thereafter, and the vapor bubble 18 starts to grow again up until time tₘₐₓ₂. This 3^{rd} phase (from time tₘᵢₙ₁ to time tₘₐₓ₂) is followed again by a collapse in the 4^{th} phase (from time tₘₐₓ₂ to time tₘᵢₙ₂). This oscillation process of the vapor bubble 18 continues, decreasing in amplitude and temporal period each time as illustrated in Fig. 6.

In various embodiments, a temporal bubble oscillation period T_{B} may be defined as the time between tₒ₁ and tₘᵢₙ₁. Temporal bubble oscillation period T_{B} varies based at least in part on the thermo-mechanical properties of the liquid 3, the shape and volume of the liquid 3 reservoir, the laser beam 5 emission profile, pulse duration, pulse energy, and so forth. Specifically, when the liquid 3 medium is contained in an endodontic access opening, e.g. in a body cavity 2 as shown in Figs. 2a, 2b 3a, 3b, and 5, the bubble's oscillation period T_{B} is prolonged, the bubble's collapse is slowed down, and no shock wave is emitted, as already explained.

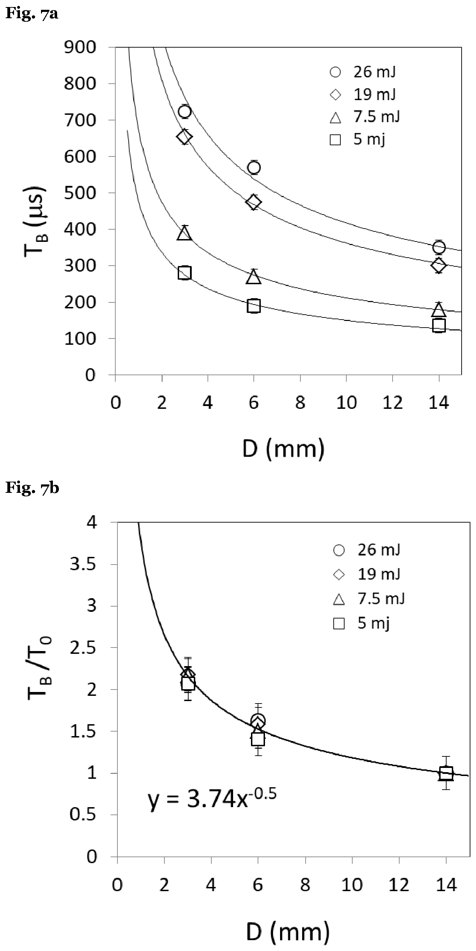

The exemplary dependence of the bubble's oscillation period T_{B} on the cavity dimensions is shown in Fig. 7a, as measured in a cylindrical model of a cavity. A LightWalker branded laser system available from Fotona, d.o.o., Slovenia was used in the measurement. The liquid 3 within the cavity 2 was water, and the laser source 4 was an Er:YAG laser with the wavelength of 2940 nm which is strongly absorbed in water. The laser pulse duration was about 50 µsec and the laser pulse energy E_{L} was 5 mJ, 7.5 mJ, 19 mJ or 26 mJ. The laser beam 5 was delivered from the laser source 4 through the Fotona Optoflex^{®} brand articulated arm 14 and the handpiece 7 (Fotona H14) to a water filled model of a root canal cavity 2 through a flat fiber tip 24 (Fotona Flat Sweeps400) with its flat surface ending 11 submersed in water to a depth h of about 3 mm. The fiber tip's diameter was 0.4 mm, and the lateral diameter (D) of the cylindrical cavity model was equal to D = 3 mm or D = 6 mm. It is to be appreciated that because of the slowing down of the bubble dynamics at D = 3 mm and D = 6 mm, no shock waves were observed when the bubble 18 imploded at t = tₘᵢₙ₁.

Referring again to Fig.7a, the depicted lines represent numerical fits to the oscillation period data using a function ${T}_{B} = K \times {D}^{- 0.5} ,$ (1) with best fits obtained with K = 475, 671, 1145 and 1320 us.mm^{0.5}, for pulse energies E_{L} = 5, 7.5, 19 and 26 mJ, correspondingly.

The dependence of T_{B} on the square root of D resembles the dependence of the oscillating period Tₗᵢₙ of a standard damped linear oscillator on a damping factor β, as ${T}_{lin} = {T}_{lino} \times {\left(1-{\left(β\times {T}_{lino}/2π\right)}^{2}\right)}^{- 0.5} ,$ (2) where Tₗᵢₙₒ is the oscillating period of the linear oscillator in the absence of damping (β = 0). Even though the oscillation dynamics of a three-dimensional bubble in a fluid within a constrained environment is much more complex than that of an ideal linear oscillator, we have thus found that the square root dependence applies to the bubble dynamics as well, providing that the oscillation period of an unconstrained bubble in a large reservoir (T₀) is assigned to a relatively large but not infinite cavity diameter of about D = 14 mm. Above this diameter, the square root approximation breaks down, and the imaginary damping factor β becomes negative. We attribute this observation to the bubble characteristics according to which the bubble oscillation period starts to increase appreciably and with the square root dependence only after the cavity diameter becomes smaller than about D = 14 mm.

According to the above, the data points for D = 14 mm in Fig. 7a represent the bubble oscillation periods as obtained in a large water reservoir. The same laser parameters and delivery system as described above were used for all liquid reservoir geometries. In the large reservoir, e.g. in a free liquid geometry, bubble oscillations can be accommodated by displacing the liquid at long distances, and therefore the oscillations were faster, with a bubble period T_{B} being up to about two times shorter than in the cylindrical cavity model. In the confined cavity model, a free expansion of the bubble laterally is not possible, and hence the water is pushed forward and backward in the root canal. Since the water obstructs the expansion of the vapor in the forward direction, the bubble grows backwards along the fiber, as can be seen from the insert in Fig. 6 at time tₘₐₓ₁. The pressure inside the bubble remains high for a long time, since it has to fight against the resistance of the water which has to be displaced in the small canal. This process delays the dynamics of expansion and implosion, and introduces additional losses compared to a free water situation. In the cavity, the lateral and forward bubble expansion is limited by the cavity wall, while the backward expansion is blocked by the fiber making the lumen of the cavity even smaller. These differences with a free water situation are considered to be the reason of a measured approximately two times longer bubble oscillation time T_{B} and in up to approximately three times smaller bubble size (V_{B}) in the cavity as compared to a large reservoir, resulting altogether in about six times slower rate of the bubble collapse (V_{B}/T_{B})/2. Consequently, no shock wave emission was detected during single pulse experiments in the confined cavity model geometry (for D = 3 and 6 mm). In turn, in the free reservoir, shock wave emission was present during the collapse of the (first) bubble without the need for a second pulse.

It is to be appreciated that the bubble implosion begins near the fiber tip where the expansion started, resulting in a separation of the bubble 18 from the fiber, as can be seen from the insert in Fig. 6 at time tₛₑₚ. Referring now to Fig. 8a and Fig. 8b, according to present invention, at first a first laser pulse pₐ and then a second laser pulse p_{b} with the same characteristics as the prior laser pulse pₐ may be delivered into the root canal model at the respective onset times tₒₐ and t_{ob} with a pulse repetition time Tₚ in between such that the second bubble 18' starts to expand at a time when the prior bubble 18 has already contracted to a certain size. This leads to a violent implosion of the prior bubble 18, and consequently to an emission of a shock wave 25 by the prior bubble 18 at the time of its collapse, even in confined geometries.

The foregoing oscillation dynamics of vapor bubbles 18 and 18' and associated relation to shock wave emission, facilitate the improved inventive system for and methods of cleaning utilizing delivery of laser pulses p, for example cleaning of root canals, drilled bone, and/or the like anatomical cavities 2 preferably with Dₐᵥₑ less than 8 mm and even more preferably with Dₐᵥₑ ≤ 6 mm. Moreover, and referring now to Figs. 8a, 8b, in various embodiments, shock wave emission can be facilitated or enhanced in confined geometries preferably with Dₐᵥₑ < 8 mm and even more preferably with Dₐᵥₑ ≤ 6 mm, and/or in highly viscous liquids by delivering a minimum of two laser pulses pₐ, p_{b} in a sequence whereas the pulse repetition time Tₚ is controlled as described herein. It is to be appreciated that the illustrations in Figs. 8a, 8b are made only for the purposes of describing the invention, and do not necessarily depict amplitudes and shapes of laser pulses, bubble volumes or shock waves, as would be observed in actual embodiments of the invention.

Fig. 8b shows an exemplary inventive laser pulse sequence with pulse durations tₚ and inventive pulse repetition time Tₚ, and the resulting dynamics of the resulting vapor bubbles and shock wave emissions. Individual pulses pₐ and p_{b} within one sequence follow each other by a pulse repetition time Tₚ. The first pulse pₐ starts at an onset time tₒₐ and generates, starting at the same onset time tₒₐ, a first vapor bubble 18. The size or volume V of the vapor bubble 18 oscillates in an expansion phase from a minimal volume at the first tₒₐ to a maximal volume Vₘₐₓ₋ₐ at a maximum volume time tₘₐₓ₁₋ₐ, and in a subsequent contraction phase from a maximal volume Vₘₐₓ₋ₐ at the maximal volume time tₘₐₓ₁₋ₐ to a minimal volume at a minimum volume time tₘᵢₙ₁₋ₐ. When within the inventive pulse sequence the pulse repetition time T_{P} is adjusted to match Tₚ₋ₒₚₜ , in other words adjusted such that an onset time t_{ob} of the subsequent laser pulse p_{b} is delivered at about the time when the first vapor bubble 18 formed by the prior laser pulse pₐ has partially collapsed as outlined herein (e.g. to a value from about 0.7 x Vₘₐₓ₋ₐ to about 0.2 x Vₘₐₓ₋ₐ, preferably from about 0.7 x Vₘₐₓ₋ₐ to about 0.3 x Vₘₐₓ₋ₐ, expediently in a range from about 0.6 x Vₘₐₓ₋ₐ to about 0.4 x Vₘₐₓ₋ₐ, and according to Fig. 8b of about 0.5 x Vₘₐₓ₋ₐ), two effects happen in parallel: As a first effect the first bubble 18 has separated from the exit component 8 and moved away downwards (Fig. 8a), in consequence of which - although the exit component 8 has not moved - the second pulse p_{b} is introduced at a location different to the location where the first vapor bubble 18 is now present at the time of introducing the second laser pulse pₐ, thereby generating the second vapor bubble 18' within the liquid 3. As a second effect the liquid pressure exerted on the collapsing prior bubble 18 by the expanding subsequent bubble 18', i.e., the bubble resulting from the subsequent laser pulse p_{b}, forces the prior bubble 18 to collapse faster, thus enabling or enhancing the emission of a shock wave 25 by the prior bubble 18, as indicated in Fig. 8a. The inventive control of the pulse repetition time Tₚ, as outlined herein, ensures that when the subsequent bubble starts substantially expanding i) the prior bubble is already in the fast collapse phase, and is therefore sensitive to the sudden additional pressure caused by the expanding subsequent bubble; and ii) in embodiments with a contact delivery of the laser energy into a liquid, the prior bubble has already substantially separated and moved away from the exit component 8, and therefore the laser energy of the subsequent laser pulse does not get absorbed within the prior bubble. However, in any case where the created vapor bubbles 18, 18' have no sufficient tendency to separate from the exit component or to otherwise change their location, and also in embodiments with a non-contact delivery, the exit component 8 or laser focal point may be spatially moved in between the pulses, for example by a scanner, as explained above, in order to avoid the laser energy of the subsequent laser pulse p_{b} to be absorbed within the prior bubble 18.

It is to be appreciated that the invention is not limited to the emission of only two subsequent pulses within a pulse set. A third pulse following a second laser pulse, and fulfilling both conditions, may be delivered resulting in an emission of a shock wave by the previous (second) bubble. Similarly, an n^{th} subsequent laser pulse will result in an emission of a shock wave by the (n-1)^{th} bubble, and so on as further laser pulses are being added to the set of pulses. The more laser pulses are delivered in one pulse set, the higher is the laser-to-shock wave energy conversion, with the energy conversion efficiency being proportional to the ratio (n-1)/n where n is the total number of laser pulses delivered in one pulse set 21 (Fig. 10).

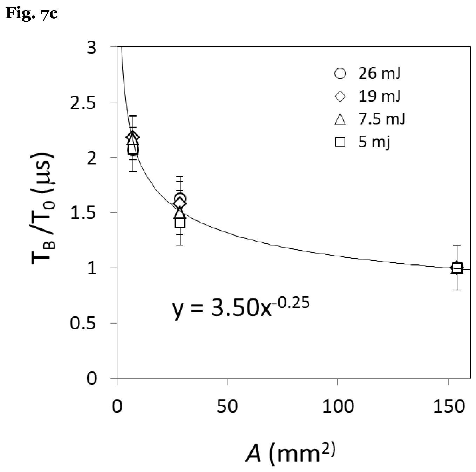

Our experiments show that the optimal repetition time (Tₚ₋ₒₚₜ) is the pulse repetition time where the subsequent bubble starts to develop during the second half of the bubble's period (T_{B}), i.e., when Tₚ = Tₚ₋ₒₚₜ = F_{S} x T_{B} where the shock wave enhancing factor (F_{S}) is in a range from about 0.6 to about 1.2, preferably in a range from about 0.75 to 0.95, and expediently in a range from about 0.8 to about 0.95. When the same device is intended to be used for cleaning differently sized cavities, containing different liquids, and with different device parameters (laser pulse energy, for example), as mentioned, this poses a challenge since as shown in Fig. 7 the bubble oscillation time (T_{B}), and consequently the optimal pulse repetition time (Tₚ₋ₒₚₜ) depend critically on these conditions, being longer, for example, for smaller reservoirs and larger laser pulse energies.

However, for most procedures there is typically only a limited set of cavity dimensions which vary from one cleaning session to another and are not under the control of the operator or the device, as opposed to "controlled" parameters, i.e., the parameters which are under the control, at least to a sufficient degree, by the device and/or the operator. Examples of controlled parameters are the wavelength of the electromagnetic source, its pulse energy and duration, or the characteristics of the employed (contact or non-contact) delivery. Therefore when keeping all the "controlled" parameters the same, the optimal repetition time (Tₚ₋ₒₚₜ) varies from cleaning session to cleaning session only as a function of the "uncontrolled" cavity dimensions. The present invention is based on the finding that particularly the lateral diameter or cross-section be advantageously used to adapt the pulse repetition time accordingly. Moreover, an aspect is also the finding that the influence of the controlled parameters, i.e., of the parameters which are at least in principle under the control of the device and the operator, can be approximately characterized by a single parameter, the "unconstrained" or "free" bubble oscillation period (T₀) representing the bubble dynamics under the conditions when the uncontrolled cavity dimensions are "infinitely" large, i.e., when the bubble dynamics is not affected by the uncontrolled spatially limited cavity dimensions. Further, it is our discovery that the optimal pulse repetition time (Tₚ₋ₒₚₜ) can be determined with sufficient accuracy solely from the known unconstrained ("free") bubble oscillation period (T₀) in combination with a characteristic cavity dimension (S), the characteristic cavity dimension S characterizing the damping influence of the constraining cavity environment (e.g. the diameter and or cross-section).

This is demonstrated in Fig. 7b that provides a different perspective on the bubble oscillation data presented in Fig. 7a. When for each laser pulse energy E_{L}, the bubble oscillation period data points T_{B} are divided by the unconstrained oscillation period Tₒ belonging to that pulse energy (i.e., by the value of T_{B} at Dₐᵥₑ = 14 mm), the obtained ratio T_{B}/Tₒ is found to be approximately independent of the laser pulse energy E_{L}, for all average diameters Dₐᵥₑ, the diameter Dₐᵥₑ thus representing a characteristic dimension S for the employed cylindrical cavity model. The full line represents the fitted function: ${T}_{B} {/ T}_{o} = {C}_{ave} \times {{D}_{ave}}^{- 0.5} ,$ (3)

With the best fit obtained for the average diameter coefficient Cₐᵥₑ = 3.74 mm^{0.5}, with the statistical coefficient of determination of R² = 0.99. Typically a fit is considered good when R² ≥ 0.7.

Similarly, and as shown in Fig. 7c, when the area of the lateral surface (A_{L}) of the cylindrical cavity is considered to represent a characteristic dimension, the best fit, represented by a full line in Fig. 7c, is obtained when the data is fitted to the function: ${T}_{B} {/ T}_{o} = {C}_{ls} \times {{A}_{ls}}^{- 0.25} ,$ (4) with the lateral area coefficient Cₗₛ = 3.50 mm^{0.5}, with R² = 0.98.

Therefore, for an "ideal" cylindrically shaped cavity, the characteristic dimension is represented either by S = D = Dₐᵥₑ or S = Aₗₛ = π x D²/4, and the optimal pulse separation (Tₚ) can be calculated for any value of the characteristic dimension using the predetermined unconstrained bubble oscillation period Tₒ characterizing the influence of the controlled parameters (such as the laser pulse energy in Figs. 7a-c), according to: ${T}_{p - opt} = {F}_{S} \times {T}_{o} \times {C}_{ave} \times {{D}_{ave}}^{- 0.5} ,$ (5) or ${T}_{p - opt} = {F}_{S} \times {T}_{o} \times {C}_{ls} \times {{A}_{ls}}^{- 0.25} ,$ (6) where Tₒ can be predetermined by a measurement and/or calculation for any combination of controlled parameters under free reservoir conditions.

It should be appreciated that in real situations the cavities may not be cylindrical but can be of any shape, an exemplary shape being illustrated in Fig. 9a. If we define the vertical direction of a cavity as the direction parallel to the direction of the delivered electromagnetic radiation (optical axis of first and/or second pulse), then the cavity's lateral surface 27 which is schematically depicted in Figs. 9a, is defined as a lateral cross section of the cavity at the location of the bubble. For the purposes of this invention, the size and shape of the lateral surface may be characterized by the lateral surface's minor (Dₘᵢₙ) and major (Dₘₐₓ) diameters. As depicted in Figs. 9a and 9b, the major diameter may be the line segment of the lateral surface that runs through the bubble and the optical axis and connects the most separated points on the cavity's inner surface. The minor diameter may be the line perpendicular to the major axis, crossing the major axis and the bubble, and extending on both sides to the cavity's inner surface. For a cylindrically shaped cavity, with lateral surface 27 being circular, Dₘᵢₙ = Dₘₐₓ = D = Dₐᵥₑ.

For an arbitrarily shaped lateral surface 27, it will be assumed that in most situations the characteristic cavity dimension S can be sufficiently well represented by either the average of the minor and major axes of the lateral surface, S = Dₐᵥₑ = (Dₘᵢₙ + Dₘₐₓ)/2. Also the cross-section area according to the present invention may be represented by the area of the lateral surface S = Aₗₛ = π x Dₘᵢₙ x Dₘₐₓ /4. It is to be noted that for a case of an elliptically shaped lateral, the minor and major diameters may correspond to the major and minor axes of such ellipse. For circularly shaped surface, the characteristic dimension may be represented by the diameter of the circle, and the cross-sectional area may be represented by Aₗₛ = π x D²/4. However, other definitions of the characteristic cavity dimension may be appropriate when so required by the type of the procedure and of the cavity shape, including potential influence of the cavity dimension in the vertical direction.

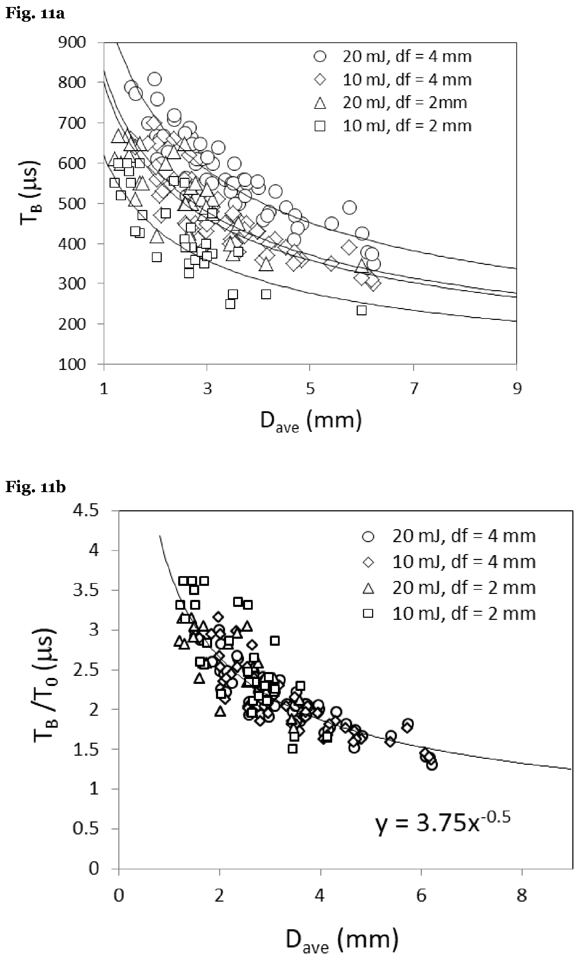

As an example, in endodontic root canal cleaning, and as shown in Figs. 10a and 10b, the endodontist makes an access opening 28 (also "access cavity" or "chamber") in the crown of the tooth, in order to enable "cleaning and shaping" of the interior of each of its root canals 29. Clinically, the size and shape of the lateral surface 27 of the access cavity 28 depends on the tooth type, the patient and also on the endodontist's skill and preference. For upper central and lateral incisors, the shape of the lateral surface is typically approximately circular. For first, second and third molars the shape of the lateral surface is quadrangular with rounded corners. And for other teeth, the shape of the lateral surface is approximately elliptical. The size and shape of the lateral surface 27 is typically described by the mesiodistal (minor) and buccolingual (major) cavity diameter, where as shown in Fig. 11b the mesiodistal cavity diameter (Dₘᵢₙ) is the diameter along the line joining the mesial and distal tooth surface, and the buccolingual cavity diameter (Dₘₐₓ) is the diameter along the line joining the buccal and lingual tooth surface.

Very roughly, the clinically encountered range from small to large minor diameters, and from small to large major diameters for different tooth types and patients is depicted in in Table 1.

**Table 1:**

| **Tooth type** | **Minor diameter Dₘᵢₙ (mm)** | | **Major diameter Dₘₐₓ (mm)** | |
|---|---|---|---|---|
| | **Small** | **Large** | **Small** | **Large** |
| Upper central incisor | 1.2 ± 0.3 | 1.9 ± 0.3 | 1.2 ± 0.3 | 1.9 ± 0.3 |
| Upper lateral incisor | 0.9 ± 0.3 | 1.6 ± 0.3 | 1.2 ± 0.3 | 1.9 ± 0.3 |
| Upper canine | 1.2 ± 0.3 | 1.9 ± 0.3 | 2.2 ± 0.3 | 2.9 ± 0.3 |
| Upper first premolar | 1.1 ± 0.3 | 1.8 ± 0.3 | 5.0 ± 0.3 | 5.7 ± 0.3 |
| Upper second premolar | 1.2 ± 0.3 | 1.9 ± 0.3 | 3.2 ± 0.6 | 4.5 ± 0.6 |
| Upper molars | 5.0 ± 1.5 | 6.6 ± 1.5 | 5.0 ± 1.5 | 6.6 ± 1.5 |
| Lower incisors | 0.5 ± 0.2 | 1.0 ± 0.2 | 1.4 ± 0.3 | 2.1 ± 0.3 |
| Lower canine | 1.2 ± 0.3 | 1.9 ± 0.3 | 2.0 ± 0.3 | 2.7 ± 0.3 |
| Lower first premolar | 1.2 ± 0.3 | 1.9 ± 0.3 | 2.2 ± 0.4 | 3.1 ± 0.4 |
| Lower second premolar | 1.1 ± 0.3 | 1.8 ± 0.3 | 2.2 ± 0.4 | 3.1 ± 0.4 |
| Lower molars | 5.0 ± 1.5 | 6.6 ± 1.5 | 5.0 ± 1.5 | 6.6 ± 1.5 |

The exemplary measured dependence of the bubble's oscillation period T_{B} on the average diameter of the lateral surface, Dₐᵥₑ = (Dₘᵢₙ + Dₘₐₓ)/2 of the endodontic access opening is shown in Fig. 11a. A LightWalker branded laser system available from Fotona, d.o.o., Slovenia was used in the measurement. The liquid 3 within the cavity 2, 28 was water, and the laser source 4 was an Er:YAG laser with the wavelength of 2940 nm which is strongly absorbed in water. The laser pulse duration was about 25 µsec and the laser pulse energy E_{L} was either 10 mJ or 20 mJ. As depicted in Figs. 11a and 11b, the laser beam 5 was delivered from the laser source 4 through the Fotona Optoflex^{®} brand articulated arm 14 and the handpiece 7 (Fotona H14) to seventy-four water filled access openings 28 of extracted teeth of different tooth types, through a flat fiber tip 24 (Fotona Flat Sweeps400) with its flat surface ending 11 submersed in water to an insertion depth h_{f} of either 2 mm or 4 mm. The fiber tip's diameter was 0.4 mm, and the average diameter of the lateral surface (Dₐᵥₑ) ranged from about 1 mm to about 6.5 mm, with Dₘᵢₙ ranging from about 1 mm to about 6 mm, and Dₘₐₓ ranging from about 1.5 mm to about 7.5 mm.

Referring again to Fig. 11a, the depicted lines represent numerical fits to the oscillation period data using the function T_{B} = Kx Dₐᵥₑ^{-0.5}, analogously to Eq. 1 and Fig. 7a. It is to be noted that the values of the numerical fits for Dₐᵥₑ = 14 mm define the unconstrained oscillation periods Tₒ. The obtained values are: K = 1010 µs.mm^{0.5} and Tₒ = 270 µs (for E_{L} = 20 mJ and h = 4 mm); K = 830 µs.mm^{0.5} and Tₒ = 214 µs (for E_{L} = 20 mJ and h = 2 mm); K = 800 µs.mm^{0.5} and Tₒ = 222 µs (for E_{L} = 10 mJ and h = 4 mm); and K = 620 µs.mm^{0.5} and Tₒ = 166 µs (for E_{L} = 10 mJ and h = 2 mm).

When analogously to Fig. 7b, the bubble oscillation period data T_{B} according to Fig. 11a, is divided by the corresponding unconstrained oscillation periods Tₒ, the obtained ratios T_{B}/Tₒ shown in Fig. 11b are found to be approximately independent of the laser pulse energy E_{L} and insertion depth h for all average diameters Dₐᵥₑ. The full line in Fig. 11b represents the result of fitting all data for all access openings and for both values of laser energy and both insertion depths to the function of Eq. 3. The best fit is obtained with T_{B}/Tₒ = Cₐᵥₑ' x Dₐᵥₑ^{-0.5}, where the average diameter coefficient for endodontic cavities is equal to Cₐᵥₑ' = 3.75 mm^{0.5} with the statistical coefficient of determination, R² ₌ 0.76, in excellent agreement with the average diameter coefficient for cylindrical cavities of C_{ave =} 3.74 mm^{0.5} (Fig. 7b).

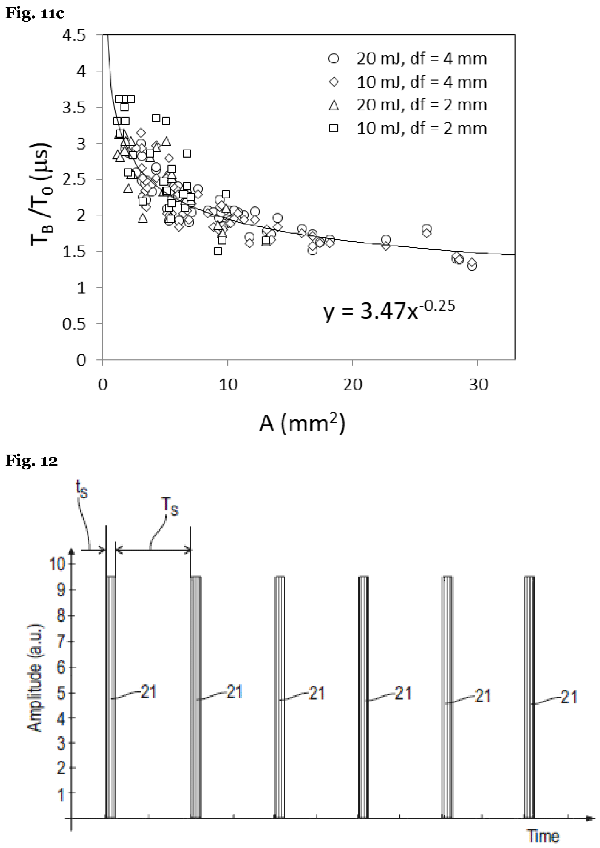

Similarly, when the bubble oscillation period data T_{B} according to Fig. 11a, is divided by the corresponding unconstrained oscillation periods Tₒ, the obtained ratios T_{B}/Tₒ shown in Fig. 11c are found to be approximately independent of the laser pulse energy E_{L} and insertion depth h for all lateral surfaces Aₗₛ = π x Dₘᵢₙ x Dₘₐₓ/4. The full line in Fig. 11c represents the result of fitting all data for all access openings and for both values of laser energy and both insertion depths to the function of Eq. 4. The best fit is obtained with T_{B}/Tₒ = Cₗₛ' x Aₗₛ^{-0.25}, where the lateral surface coefficient for endodontic cavities (Cₗₛ') is equal to Cₗₛ' = 3.47 mm^{0.5} with R² = 0.76, also in excellent agreement with the lateral surface coefficient of Cₗₛ = 3.50 mm^{0.5} for the "ideal" cylindrically shaped cavity (Fig. 7c).

Therefore, for the embodiments of our invention where the size and shape of the lateral surface 27 represent the most significant uncontrolled varying cavity size influencing the bubble dynamics, the pulse separation times which are about optimal for most of the cavities can be taken to be the same as for an ideal cylindrical cavity, and are thus determined according to Eq. 3 using Dₐᵥₑ = (Dₘᵢₙ + Dₘₐₓ)/2, and Cₐᵥₑ = 3.74 mm^{0.5} or according to Eq. 4 using Aₗₛ = π x Dₘᵢₙ x Dₘₐₓ/4, and Cₗₛ = 3.50 mm^{0.5}.

However, for some procedures where there exists a sufficiently strong correlation between sizes of Dₘᵢₙ and Dₘₐₓ, the minor or major diameter alone can represent a statistically significant characteristic dimension. For example, for the endodontic data according to Fig. 11a, a good fit was obtained also with: ${T}_{B} {/ T}_{o} = {C}_{min} \times {{D}_{min}}^{- 0.5} ,$ (7)
where Cₘᵢₙ = 3.45 mm^{0.5} with R² = 0.75; and ${T}_{B} {/ T}_{o} = {C}_{max} \times {{D}_{max}}^{- 0.5} ,$ (8)
where Cₘₐₓ = 3.95 mm^{0.5} with R² = 0.70, resulting in ${T}_{p - opt} = {F}_{S} \times {T}_{o} \times {C}_{min} \times {{D}_{min}}^{- 0.5} ,$ (9)
and ${T}_{p - opt} = {F}_{S} \times {T}_{o} \times {C}_{max} \times {{D}_{max}}^{- 0.5} .$ (10)

It is noted that the ranges indicated above for parameters K_{D} and K_{A} approximately correspond to the ranges of the products F_{S} x Cₐᵥₑ, Fs x Cₘᵢₙ, Fs x Cₘₐₓ, and of the product F_{S} x Cₗₛ respectively.

Further, it is also within the present scope that more specific ranges of K_{D} relate to ranges of each of F_{S} x Cₐᵥₑ, F_{S} x Cₘᵢₙ, and/or F_{S} x Cₘₐₓ individually, wherein Fₛ varies within the preferred ranges as outlined herein and Dₐᵥₑ, Dₘᵢₙ, Dₘₐₓ would be used as D (in the formula K_{D} x Tₒ x D^{-0.5}). Similarly, the ranges specified for K_{D} may also be used instead of those for K_{A} (in K_{A} x Tₒ x A^{-0.25}), providing that the units for K_{D} (in mm^{0.5}) are replaced by units for K_{A} (in mm^{0.25}).

In one of the exemplary embodiments an Er:YAG laser was used to perform enhanced irrigation of the access opening and root canals, where the set of relevant controlled parameters includes laser pulse energy (E_{L}), laser pulse duration (tₚ), the fiber tip geometry: a flat fiber with output shape 11 or a pointed fiber with output shape 13, the fiber tip's diameter D, and the depth of insertion h. The corresponding values of the bubble oscillation period for an infinitely large lateral surface 27 of the endodontic access cavity 28, as obtained using the same fitting technique as presented in Fig. 11a, are shown in Table 2. The data presents an embodiment where the laser beam 5 extends substantially across the whole cross section of the fiber tip 23.

**Table 2:**

| **Fiber tip geometry** | | | **Flat** | | | | **Pointed** | |
|---|---|---|---|---|---|---|---|---|
| **Fiber tip diameter** | | | **300 µm** | **400 µm** | **500 µm** | **600 µm** | **400 µm** | **600 µm** |
| **tₚ(µs)** | **E_{L}(mJ)** | **h (mm)** | **Tₒ(µs)** | **Tₒ(µs)** | **Tₒ(µs)** | **Tₒ(µs)** | **Tₒ(µs)** | **Tₒ(µs)** |
| 25 | 10 | 2 | 161 | 166 | 153 | 146 | 166 | 147 |
| 25 | 20 | 2 | 202 | 214 | 199 | 190 | 215 | 188 |
| 25 | 10 | 4 | 216 | 222 | 204 | 195 | 225 | 196 |
| 25 | 20 | 4 | 254 | 270 | 251 | 240 | 271 | 237 |
| 50 | 10 | 2 | 145 | 141 | 137 | 134 | 134 | 123 |
| 50 | 20 | 2 | 183 | 187 | 168 | 168 | 207 | 170 |
| 50 | 10 | 4 | 193 | 188 | 184 | 179 | 202 | 165 |
| 50 | 20 | 4 | 231 | 235 | 212 | 212 | 261 | 215 |

It is to be appreciated that the values of Tₒ for E_{L}, tₚ, h, and D which are not presented in Table 2, can be approximately obtained by constructing new data points within and as well below and above the range of the discrete set of values depicted in Table 2, using a linear or a suitable higher order interpolation or fitting method. In some examples, the control unit may be adapted to interpolate such values for Tₒ based on two or more known values of Tₒ. For example, a table including one or more values of Table 2 may be stored in a storage device as outlined above, and, depending on the controlled parameters, the control unit may select a value of Tₒ from the table and/or interpolate a suitable value based on two or more values stored in the table. Additionally or alternatively, the operator of the apparatus may be provided with the table, and he/she may then enter a suitable value for Tₒ via the user interface.

The control unit may be adapted to control the pulse repetition as a function of the unconstrained oscillation period Tₒ of the first vapor bubble, which may depend on the wavelength of the radiation beam, and/or the energy of the first laser pulse (pₐ), and/or the pulse duration of the first pulse (tₚ), and/or the exit component 8 and/or the insertion depth, e.g. according to Table 2 or interpolations thereof, e.g. using one or more of the Eqs. 5, 6, 9 or 10, such that the interaction between the first vapor bubble 18 and the second vapor bubble 18' generates a shock wave within the liquid 3.

In one of the preferred embodiments, the apparatus, e.g. implemented as a cleaning system configured for cleaning of cavities, e.g., endodontic access opening cavities, filled with liquid. A cavity may have a lateral surface characterized by a minor and/or major inner diameter (Dₘᵢₙ, Dₘₐₓ), that may vary from cavity to cavity. The cleaning system may comprise an electromagnetic radiation system, e.g. a laser system, wherein the electromagnetic radiation system is adapted to be operated in pulsed operation with at least one pulse set (21) containing at least two individual pulses (p) having each an individual pulse energy, wherein within the pulse set (21) a first pulse (pₐ) of the pulses (p), having a pulse duration (tₚ) and pulse energy (E_{L}), is followed by a second pulse (p_{b}) of the pulses (p) with a pulse repetition time (Tₚ), wherein the pulses are adapted to generate a first vapor bubble (18) within the liquid (3) by means of the corresponding first pulse (pₐ) and to generate a second vapor bubble (18') within the liquid (3) by means of the
corresponding second pulse (p_{b}). The pulse repetition time is controlled, e.g. by a control unit, based on the unconstrained oscillation period Tₒ of the first vapor bubble as a function of the wavelength of the radiation beam, and/or the energy of the first laser pulse (pₐ), and/or the pulse duration of the first pulse (tₚ), and/or the exit component 8 and/or the insertion depth (h) according to Table 2 or by some other data characterizing the influence of the above said parameters. Preferably, the control unit 22 is adapted to adjust the pulse repetition time (Tₚ) as a function of the unconstrained oscillation period Tₒ of the first vapor bubble and of the cavity minor inner diameter (Dₘᵢₙ) and/or major inner diameter (Dₘₐₓ), using at least one of the Eqs. 5, 6, 9 or 10, such that the interaction between the first vapor bubble (18) and the second vapor bubble (18') generates a shock wave within the liquid (3).

Fig. 12 shows in a schematic diagram an exemplary temporal course of pulse sets 21 according to the invention. In this connection, the course of the amplitude of the pulse sets 21 is illustrated as a function of time. The pulse sets 21 follow one another along one single optical path within the laser system 1 with a temporal pulse set spacing T_{S} being the temporal difference between the end of one pulse set 21 and the beginning of the next pulse set 21. The temporal pulse set spacing T_{S} is expediently 10 ms ≤ T_{S} ≤ 500 ms, advantageously 10 ms ≤ T_{S} ≤ 100 ms, and is in the illustrated embodiment of the inventive method approximately 10 ms. The lower temporal limit for temporal set spacing T_{S} of 10 ms is set in order to allow sufficient time for the laser active material, such as, for example, a flash-lamp pumped laser rod, to cool off during the time between subsequent pulse sets 21. The individual pulse sets 21 have a temporal set length t_{S} of, for example, approximately 2 ms. Depending on the number of individual pulses p provided infra the value of the temporal set length t_{S} can vary. The maximal number of pulse sets 21, and correspondingly the maximal number of individual pulses p, that may be delivered during a cleaning session is limited at least by the maximal delivered cumulative energy below which the temperature increase of the liquid 3 does not exceed an allowed limit.

It is to be appreciated that, the bubble oscillation periods T_{B} as defined by Eqs. 3, 4, 7, 8 represent only average oscillation periods, and that in practice the oscillation periods may be spread around those average values of the oscillation periods T_{B}, as demonstrated in exemplary embodiments depicted in Figs.11b and 11c. Therefore, the Tₚ₋ₒₚₜ as calculated for the average bubble oscillation periods according to Eqs. 5 and 6, or 9 and 10 may not be perfectly optimal to generate a shock wave within a particular liquid-filled cavity.

This may be solved in yet another embodiment, where, in order to facilitate automatic adjustability of the pulse repetition time Tₚ to an expected spread of the bubble oscillation period around the expected average oscillation period (e.g. corresponding to minor deviations due to the specifics of the cavity geometries), the apparatus (e.g. laser system 1) is configured with a laser source 4 having a (automatically) variable, "sweeping" pulse generation. In this manner, the shock wave emission may be automatically optimized for particular cavity dimensions and shapes and or for particular controlled parameters. The general idea of the inventive sweeping technique is to generate multiple pairs of first and second bubbles 18, 18' such, that the time difference between the onset time t_{ob} of the second vapor bubble 18' and the onset time tₒₐ of the first vapor bubble 18 (Fig. 8b) is repeatedly varied in a sweeping manner. By varying said time difference it is made sure, that at least one pair of bubbles 18, 18' matches the required timing, as with the first and second bubbles 18, 18' of Fig. 8b, and thus emitting at least one shock wave 25 (Fig. 8a) during each sweeping cycle. By repeatedly performing such sweeping cycles, the generation of shock waves 25 may be repeated to an extent until the desired irrigation goal is achieved.

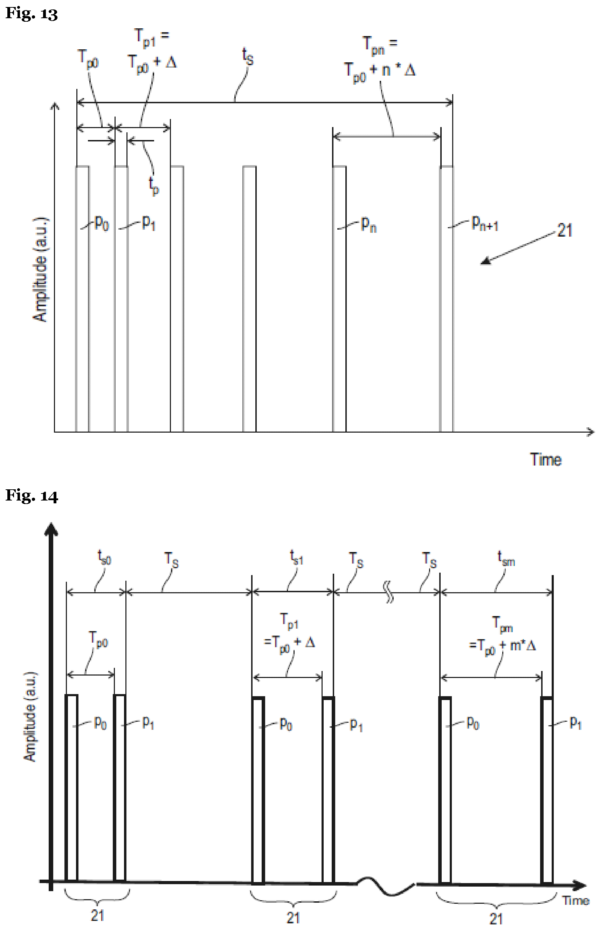

Fig. 13 shows an enlarged detail illustration of the diagram according to Fig. 12 in the area of an individual pulse set 21. Each pulse set 21 has expediently at least two and maximally 20 individual pulses p, advantageously two to eight individual pulses p, and preferably two to four individual pulses p, and in the illustrated embodiment according to Fig. 13 there are six individual pulses p. Maintaining the aforementioned upper limit of the number of individual pulses p per pulse set 21 avoids overheating of the laser active material. The individual pulses p have a temporal pulse duration tₚ and follow one another along one single optical path within the laser system in a pulse repetition time Tₚ, the pulse repetition time T_{P} being the time period from the beginning of one single pulse p to the beginning of the next, subsequent pulse p.

The pulse duration tₚ is for weakly absorbed wavelengths in the range of ≥ 1 ns and < 85 ns, and preferably ≥ 1 ns and ≤ 25 ns. The lower temporal limit of the pulse duration tₚ for weakly absorbed wavelengths ensures that there are no shock waves created in the liquid 3 during the vapor bubble 18 expansion. And the upper pulse duration tₚ limit for weakly absorbed wavelengths ensures that the laser pulse power is sufficiently high to generate optical breakdown in the liquid.

For highly absorbed wavelengths, the pulse duration tₚ is in the range of ≥ 1 µs and < 500 µs, and preferably of ≥ 10 µs and < 100 µs. The lower temporal limit for highly absorbed wavelengths ensures that there is sufficient pulse energy available from a free-running laser. And the upper pulse duration limit for highly absorbed wavelengths ensures that the generated heat does not spread via diffusion too far away from the vapor bubble, thus reducing the laser-to-bubble energy conversion efficiency. Even more importantly, the upper pulse duration limit ensures that laser pulses are shorter than the vapor bubble rise time, tₘₐₓ₁ - tₒ₁, in order not to interfere with the bubble temporal oscillation dynamics. In Fig. 10, the amplitude of the laser beam or of its individual pulses p is schematically plotted as a function of time wherein the temporal course of the individual pulses p, for ease of illustration, are shown as rectangular pulses. In practice, the pulse course deviates from the schematically shown rectangular shape of Fig. 13.

Referring now to Fig. 13, a first exemplary shock wave emission enhancing pulse (SWEEPS) set 21 according to the invention is proposed, wherein the pulse repetition time T_{P} is varied or "swept" in discreet positive or negative steps Δ from an initial pulse period Tₚₒ to a final pulse period Tₚₘ, preferably +- across a range from Tₚₒ = Tₚ₋ₒₚt - δ₁ to Tₚₘ = Tₚ₋ₒₚₜ + δ ₂ (or from Tₚₒ = Tₚ₋ₒₚₜ + δ ₂ to Tₚₘ = Tₚ₋ₒₚₜ - δ ₁ in the case of a negative Δ), where δ ₁ and δ ₂ are each preferably in a range from 10 to 300 µsec, even more preferably in a range from 20 to 75 µsec, and expediently in a range from 25 to 75 µsec. In a preferred embodiment δ ₁ = δ ₂. By using this inventive pulse repetition sweeping technique, it is ensured that at least one pair of pulses p within the number of multiple pulses pₒ to pₙ, pₙ₊₁ of Fig. 13 matches the required pulse repetition rate, thereby resembling the first and second pulses pₐ, p_{b} of Fig. 8b with the required adjusted and optimal pulse repetition time T_{P} = Tₚ₋ₒₚₜ in between, and thus generating at least one fitting pair of bubbles 18, 18' (Fig. 8b) for emitting at least one shock wave during each sweeping cycle. Notably, by sweeping within a small range around the estimated optimum pulse repetition time (e.g. determined from the "unconstrained bubble oscillation period" and a "diameter" of the cavity), it may be ensured that the true optimum pulse repetition time is achieved with certainty for the specific cavity at hand.

The pulse repetition time T_{P} may be "swept" within each pulse set 21 as exemplarily shown in Fig. 13 where the pulse repetition time Tₚ is discretely swept from pulse pₒ to pulse pₙ₊₁ by changing the pulse repetition time Tₚ from pulse to pulse by an additional discreet temporal step Δ, while multiple pulse sets 21 of such or similar kind may follow one another. In the illustrated embodiment of the inventive method, pulse sets 21 consisting of six pulses pₒ to pₙ₊₁ are shown, but pulse sets 21 with a larger or smaller number of pulses p may be used as well.

Alternatively, as a second preferred sweeping pattern, a number of m pulse sets 21 may be applied, wherein the pulse repetition time T_{P} may be varied or "swept" from pulse set 21 to pulse set 21 as exemplarily shown in Fig. 14: The pulse repetition time Tₚ is discretely swept from pulse set 21 to pulse set 21 by starting at an initial repetition time Tₚₒ, and then changing the pulse repetition time Tₚ from pulse set 21 to pulse set 21, by a discreet temporal step Δ to a final repetition time Tₚₘ. The sweeping cycle may be restarted each time the whole sweeping range has been covered. In the embodiment of the inventive method illustrated in Fig. 11, pulse sets consisting of two pulses p₀, p₁ are shown, but pulse sets with a larger number of pulses p may be used as well. In any case the same effect as with the sweeping pattern of Fig. 10 can be achieved: At least one pair of pulses p₀, p₁ within the number of m pulse sequences 21 of Fig. 14 matches the required pulse repetition rate, thereby resembling the first and second pulses pₐ, p_{b} of Fig. 8b with the required adjusted and optimal pulse repetition time T_{P} = Tₚ₋ₒₚₜ in between, and thus emitting at least one shock wave during each sweeping cycle.

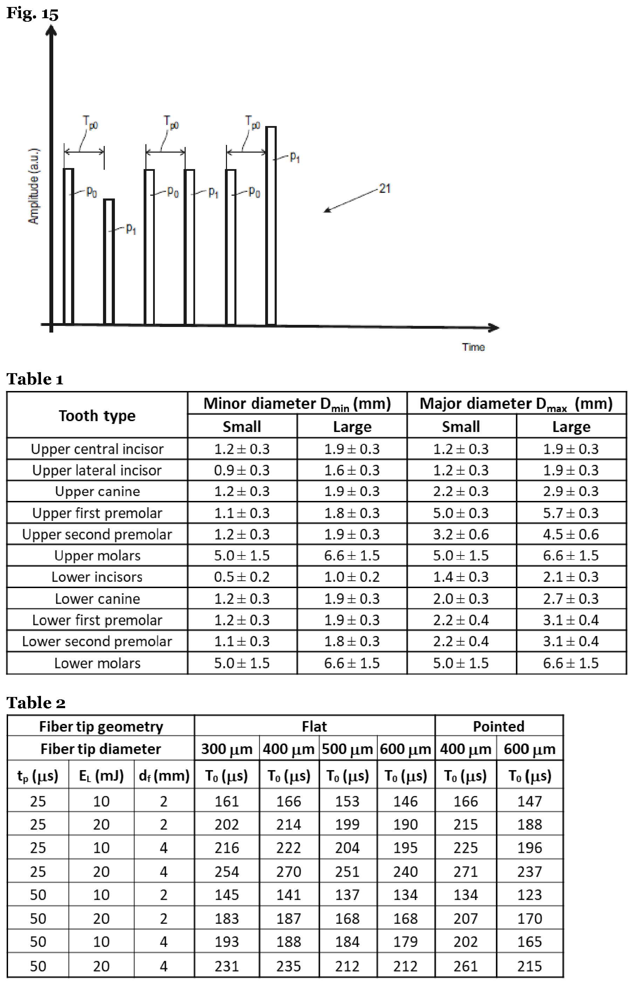

A further preferred, third sweeping pattern is schematically depicted in Fig. 15: One pulse set 21 contains multiple pairs of two pulses p₀, p₁, wherein a subsequent pulse p₂ of each pair follows a corresponding initial pulse p₁, and wherein the pulse repetition times Tₚ within all pairs is kept constant. However, from pair of pulses p₀, p₁ to a subsequent pair of pulses p₀, p₁, the pulse energy of each second pulse p₁ is varied in a sweeping manner. In the shown example the pulse energy is increased from pair to pair by a certain delta. On the other hand, an energy decrease may be applied as well. Such pulse energy sweeping is based on the finding, that the lower the second pulsed p₁ energy is, the longer it will take the second bubble 18' (Fig. 8a, 8b) to develop appreciably to influence the first bubble's 18 collapse, and vice versa. This way it can again be achieved, that at least one pair of bubbles 18, 18' matches the required timing, as with the first and second bubbles 18, 18' of Fig. 8b, and thus emitting at least one shock wave 25 (Fig. 8a) during each sweeping cycle. Additionally or alternatively, also the energy of the first pulse may be swept, similarly as described with reference to the second pulse.

A combined SWEEP method may be used as well, where the pulse repetition time T_{P} is "swept" within pulse sets 21 from one pulse p to another, and/or from pulse set 21 to pulse set 21. Furthermore, the sweeping pulse energy of Fig. 15 may be combined with the sweeping pulse repetition times T_{P} of Fig. 13 and/or of Fig. 14.

In order to facilitate improved adjustability and/or control, in various embodiments, the laser system 1 may be configured with a laser source 4 having variable pulse parameters, e.g. a variable pulse rate or repetition time, a variable pulse energy, a variable pulse set rate, and/or a variable temporal pulse set length t_{S} of the pulse set 21. In this manner, the shock wave emission may be optimized for particular cavity dimensions and shape, and also for a particular placement of the fiber tip or positioning of the laser focus in the different locations relative to the cavity. Namely, the placement of the fiber tip or positioning of the laser focus relative to the cavity may affect the properties of the bubble oscillations and shock wave emission. In one of the embodiments, a centering system may be used to center the fiber tip relative to the walls of the cavity, and/or to center the fiber tip near the entrance, or bottom of the cavity, or near an occlusion within the cavity.

The bubble oscillation period T_{B} may for example vary from about 10 µs to about 3000 µs, based at least in part on the thermo-mechanical properties of the liquid 3, the shape and volume of the liquid reservoir, the laser wavelength, beam emission profile, configuration of the head, and so forth. Accordingly, when the pulse repetition time T_{P} will be adjusted to approximately match Tₚ₋ₒₚₜ (e.g. adjusted to a range between approximately 60% T_{B} and approximately 95% T_{B}), the pulse repetition rate F_{P}, will be in the range from about 0.35 kHz to about 167 kHz, such that the laser source 1 may be adapted accordingly.

The laser pulse energy E_{L}, according to the invention, may be fixed for all pulses within a pulse set 21. In certain embodiments, however, the energy of the subsequent pulse may be adjustable to automatically gradually decrease, for example linearly or exponentially, from pulse to pulse within each set 21. This approach may be especially advantageous for pulse sets with a pulse number of n = 2, where the energy E_{L} of the second pulse p_{b} may be lower than that of the first pulse pₐ, since the function of the second bubble 18' is only to create an additional pressure on the collapsing bubble 18 during the initial expansion phase of the bubble 18'.

Alternatively, the laser pulse energy E_{L} may be adjustable to gradually increase from pulse to pulse within a pulse set 21, in order to increase even further the pressure of the subsequent bubbles on the prior bubbles.

Additionally or alternatively, in one of the embodiments of our invention, the laser system may comprise a feedback system to determine the bubble dynamics and feed it back to the control unit such as to control deviations of the pulse repetition time around the estimated optimum frequency to optimize shock wave generation.

For example, the amount of shock waves generated may be measured by sweeping, and the feedback system may be adapted to control the pulse repetition frequency such as to maximize the shock wave generation. In other words, a closed control loop control for automatically delivering a subsequent laser pulse at the appropriate Tₚ₋ₒₚₜ is formed. In other examples, as a result of the measured amount of shock waves, the laser pulse repetition time Tₚ might be manually adjusted by the user to be approximately equal to Tₚ₋ₒₚₜ.

Several irrigants for the endodontic cleaning are available, and include sodium hypochlorite (NaOCl), chlorhexidine gluconate, alcohol, hydrogen peroxide and ethylenediaminetetraacetic acid (EDTA). However, in one of the preferred embodiments only water may be used instead of a potentially toxic irrigant since the generation of shock waves according to our invention reduces or eliminates the need for the use of chemicals.

It will be appreciated that, while the foregoing example methods are directed to cleaning of root canals and/or bone cavities, in accordance with principles of the present disclosure, similar methods and/or systems may be utilized to clean other body tissues, for example periodontal pockets, and/or the like. The method may be also used to clean selected small surfaces of electronic and precision mechanical components during manufacturing, maintenance and servicing, especially when it is not desirable or possible to expose the whole electronic or other component to a standard cleaning or irrigation procedure.

While the principles of this disclosure have been shown in various embodiments, many modifications of structure, arrangements, proportions, the elements, materials and components, used in practice, which are particularly adapted for a specific environment and operating requirements may be used without departing from the principles and scope of this disclosure. These and other changes or modifications are intended to be included within the scope of the present disclosure and may be expressed in the following claims.

The present disclosure has been described with reference to various embodiments. However, one of ordinary skill in the art appreciates that various modifications and changes can be made without departing from the scope of the present disclosure. Accordingly, the specification is to be regarded in an illustrative rather than a restrictive sense, and all such modifications are intended to be included within the scope of the present disclosure. Likewise, benefits, other advantages, and solutions to problems have been described above with regard to various embodiments. However, benefits, advantages, solutions to problems, and any element(s) that may cause any benefit, advantage, or solution to occur or become more pronounced are not to be construed as a critical, required, or essential feature or element of any or all the claims.

Systems, methods and computer program products are provided. In the detailed description herein, references to "various embodiments", "one embodiment", "an embodiment", "an example embodiment", etc., indicate that the embodiment described may include a particular feature, structure, or characteristic, but every embodiment may not necessarily include the particular feature, structure, or characteristic.

Moreover, such phrases are not necessarily referring to the same embodiment. Further, when a particular feature, structure, or characteristic is described in connection with an embodiment, it is submitted that it is within the knowledge of one skilled in the art to affect such feature, structure, or characteristic in connection with other embodiments whether or not explicitly described. After reading the description, it will be apparent to one skilled in the relevant art(s) how to implement the disclosure in alternative embodiments.

As used herein, the terms "comprises," "comprising," or any other variation thereof, are intended to cover a non-exclusive inclusion, such that a process, method, article, or apparatus that comprises a list of elements does not include only those elements but may include other elements not expressly listed or inherent to such process, method, article, or apparatus. Also, as used herein, the terms "coupled," "coupling," or any other variation thereof, are intended to cover a physical connection, an electrical connection, a magnetic connection, an optical connection, a communicative connection, a functional connection, and/or any other connection. When language similar to "at least one of A, B, or C" is used in the claims, the phrase is intended to mean any of the following: (1) at least one of A; (2) at least one of B; (3) at least one of C; (4) at least one of A and at least one of B; (5) at least one of B and at least one of C; (6) at least one of A and at least one of C; or (7) at least one of A, at least one of B, and at least one of C.

The following numbered embodiments are included in the disclosure:
1. An apparatus (1) for applying pulses of electromagnetic radiation to a cavity (2) filled with a liquid (3), comprising:
   a source (4, 4') for generating a first pulse and a second pulse of electromagnetic radiation;
   a control unit (22) adapted to control a time between the first pulse and the second pulse as a function of a diameter D and/or a cross-sectional area of the cavity (2).
2. The apparatus according to embodiment 1, further comprising a user interface (30) for receiving information on the diameter and/or cross-sectional area of the cavity.
3. The apparatus according to embodiment 1 or 2, further comprising means (9) for determining the diameter and/or cross-sectional area of the cavity.
4. The apparatus according to any of embodiments 1-3, wherein the control unit is further adapted to control the time between the first pulse and the second pulse such that it varies with the inverse root of the diameter of the cavity.
5. The apparatus according to any of embodiments 1-4, wherein the control unit is further adapted to control the time between the first pulse and the second pulse as a function of a predetermined parameter that is specific to at least an energy of the first pulse and/or to the liquid.
6. The apparatus according to embodiment 5, wherein the predetermined parameter is independent of the geometry of the cavity.
7. The apparatus according to embodiment 5 or 6, wherein the predetermined parameter corresponds to an unconstrained oscillation period T₀ of a bubble that would be generated by the first pulse in an infinitely large cavity filled with the liquid.
8. The apparatus according to any of embodiments 5-7, wherein the control unit is adapted to determine the predetermined parameter by accessing a data storage device of the apparatus and/or a remote data storage device.
9. The apparatus according to any of embodiments 5-8, wherein the control unit is adapted to control the time between the first pulse and the second pulse such that it is proportional to the predetermined parameter.
10. The apparatus according to any of embodiments 5-9, wherein the control unit is adapted to control the time according to the function K_{D} x T₀ x D^{-0.5}, wherein K_{D} is selected from the range 2 mm^{0.5} to 4.8 mm^{0.5}, preferably from the range 2.5 mm^{0.5} to 3.8 mm^{0.5}, and more preferably from the range 2.7 mm^{0.5} to 3.8 mm^{0.5}.
11. The apparatus according to any of embodiments 1-10, wherein the first pulse is adapted to generate a first bubble within the liquid, and the second pulse is adapted to generate a second bubble within the liquid, such that a shock wave is generated within the liquid.
12. The apparatus according to any of embodiments 1-11, further comprising means (26) for providing the liquid to the cavity.
13. The apparatus according to any of embodiments 1-12, wherein the control unit is adapted to determine an optimal time Tₚ₋ₒₚₜ and adapted to vary times between subsequent pairs of pulses within the range from Tₚ₋ₒₚₜ - δ₁ to Tₚ₋ₒₚₜ + δ₂, wherein δ₁ and δ₂ are selected from the range 10 µs to 300 µs, preferably from 20 µs to 75 µs and more preferably from 25 µs to 75 µs.
14. A method for applying pulses of electromagnetic radiation to a cavity filled with a liquid, comprising the steps of:
   generating a first pulse and a second pulse of electromagnetic radiation;
   controlling a time between the first pulse and the second pulse as a function of a diameter D and/or a cross-sectional area of the cavity.
15. The method according to embodiments 14, further including controlling the time between the first pulse and the second pulse as a function of a predetermined parameter that is specific to at least an energy of the first pulse and to the liquid.

## Claims

1. An apparatus (1) for applying pulses of electromagnetic radiation to a cavity (2) filled with a liquid (3), comprising:
a source (4, 4') for generating a first pulse and a second pulse of electromagnetic radiation; and
a control unit (22) adapted to control a time between the first pulse and the second pulse as a function of a diameter D and/or a cross-sectional area of the cavity (2), wherein the control unit (22) is pre-calibrated with a pulse repetition time as a function of the diameter D and/or the cross-sectional area of the cavity.

2. The apparatus according to claim 1, wherein the control unit (22) is pre-calibrated with an optimum pulse repetition time as a function of the diameter D and/or the cross-sectional area of the cavity (2).

3. The apparatus according to claim 1 or 2, wherein the pre-calibrated pulse repetition time is stored in a data storage device of the apparatus and/or a remote data storage device, preferably in the form of a look-up table or a database.

4. The apparatus according to any one of claims 1 to 3, further comprising a user interface (30) for receiving information on the diameter and/or cross-sectional area of the cavity.

5. The apparatus according to any one of claims 1-4, wherein the control unit (22) is adapted to receive a user input indicative of a tooth type and/or a cavity type, and, based on the tooth type and/or cavity type, to determine a minor and/or major diameter for the tooth type and/or the cavity type.

6. The apparatus according to claim 5, wherein the minor diameter and/or the major diameter associated with the tooth type and/or cavity type is retrieved by accessing a look-up table or a database associating a tooth type and/or cavity type with a minor diameter and/or a major diameter.

7. The apparatus according to claim 5 or 6, wherein the control unit (22) is adapted to distinguish two tooth types, wherein a first minor diameter and/or a first major diameter is associated with molar teeth, and a second minor diameter and/or a second major diameter is associated with all other teeth.

8. The apparatus according to any of claims 1-7, wherein the control unit is further adapted to control the time between the first pulse and the second pulse such that it varies with the inverse root of the diameter of the cavity.

9. The apparatus according to any of claims 1-8, wherein the control unit is further adapted to control the time between the first pulse and the second pulse as a function of a predetermined parameter that is specific to at least an energy of the first pulse and/or to the liquid, preferably wherein the predetermined parameter is independent of the geometry of the cavity; and/or preferably wherein the predetermined parameter corresponds to an unconstrained oscillation period T₀ of a bubble that would be generated by the first pulse in an infinitely large cavity filled with the liquid.

10. The apparatus according to claim 9, wherein the control unit is adapted to control the time between the first pulse and the second pulse such that it is proportional to the predetermined parameter; and/or
wherein the predetermined parameter corresponds to an unconstrained oscillation period T₀, and the control unit is adapted to control the time according to the function K_{D} x T₀ x D^{-0.5}, wherein K_{D} is selected from the range 2 mm^{0.5} to 4.8 mm^{0.5}, preferably from the range 2.5 mm^{0.5} to 3.8 mm^{0.5}, and more preferably from the range 2.7 mm^{0.5} to 3.8 mm^{0.5}.

11. The apparatus according to any of claims 1-10, wherein the first pulse is adapted to generate a first bubble within the liquid, and the second pulse is adapted to generate a second bubble within the liquid, such that a shock wave is generated within the liquid; and/or
further comprising means (26) for providing the liquid to the cavity; and/or wherein the control unit is adapted to determine an optimal time Tₚ₋ₒₚₜ and adapted to vary times between subsequent pairs of pulses within the range from Tₚ₋ₒₚₜ - δ₁ to Tₚ₋ₒₚₜ + δ₂, wherein δ₁ and δ₂ are selected from the range 10 µs to 300 µs, preferably from 20 µs to 75 µs and more preferably from 25 µs to 75 µs.

12. A method for applying pulses of electromagnetic radiation to a cavity filled with a liquid, comprising the steps of:
generating a first pulse and a second pulse of electromagnetic radiation; and
controlling a time between the first pulse and the second pulse as a function of a diameter D and/or a cross-sectional area of the cavity, using a pre-calibrated pulse repetition time as a function of the diameter D and/or the cross-sectional area of the cavity.

13. The method according to claim 12, further comprising receiving a user input indicative of a tooth type and/or a cavity type, and, based on the tooth type and/or cavity type, determining a minor and/or major diameter for the tooth type and/or cavity type.

14. The method according to claim 13, wherein the minor and/or major diameter associated with the tooth type and/or cavity type is retrieved by accessing a look-up table or database associating a tooth type and/or cavity type with a minor diameter and/or a major diameter.

15. The method according to any of claims 12-14, wherein two tooth types are distinguished, a first minor diameter and/or a first major diameter being associated with molar teeth, and a second minor diameter and/or a second major diameter being associated with all other teeth.
